# EUROPEAN PATENT SPECIFICATION

(11) **EP 3 980 029 B1**
(45) Date of publication and mention of the grant of the patent: **11.10.2023**
(21) Application number: 20751469.6
(22) Date of filing: 09.06.2020
(51) Int. Cl.: A61K 31/715, C08B 37/08, C08L 5/08

(54) **MEANS FOR USE IN PREPARATION OF HYDROGEL BASED ON HYDROXYPHENYL DERIVATIVE OF HYALURONAN, METHOD OF HYDROGEL PREPARATION AND USE THEREOF**
MITTEL ZUR VERWENDUNG IN DER HERSTELLUNG VON HYDROGEL AUF BASIS VON HYDROXYPHENYLDERIVAT VON HYALURONAN, VERFAHREN ZUR HYDROGELHERSTELLUNG UND VERWENDUNG DAVON
MOYEN DESTINÉ À ÊTRE UTILISÉ DANS UNE PRÉPARATION D'HYDROGEL À BASE DE DÉRIVÉ HYDROXYPHÉNYLE D'HYALURONANE, PROCÉDÉ DE PRÉPARATION D'HYDROGEL ET UTILISATION ASSOCIÉE

(30) Priority: 10.06.2019 CZ 20190360
(43) Date of publication of application: 13.04.2022
(73) Proprietor: Contipro A.S., 56102 Dolni Dobrouc (CZ)
(72) Inventor: PRAVDA, Martin, 56544 Sruby (CZ); KOVAROVA, Lenka, 73801 Frydek-Mistek (CZ); SCIGALKOVA, Ivana, 77900 Olomouc, Neredin (CZ); BYSTRONOVA, Julie, 73506 Karvina, Nove Mesto (CZ); TOROPISYN, Evgeniy, 10100 Praha 10, Michle (CZ); VELEBNY, Vladimir, 56401 Zamberk (CZ)
(74) Representative: Dvorakova, Martina
(86) International application number: PCT/CZ2020/050043
(87) International publication number: WO 2020/249143

(56) References cited:
- EP-A2- 2 448 610
- WO-A1-2009/148405
- WO-A1-2015/054125

## Description

### Field of the invention

The present invention relates to a means for use in a preparation of a hydrogel based on a hydroxyphenyl derivative of hyaluronan (HATA) with content of blood or transfusion preparations with content of fibrinogen, that may serve as biomaterials usable in the field of tissue engineering, regenerative medicine and further medicinal fields. The present invention further relates to the hydrogel based on the hydroxyphenyl derivative of hyaluronan and the method of preparation and the use thereof.

### State of the art

Polymer hydrogels are an important group of materials that are used in the development of medicinal means in the field of tissue engineering and regenerative medicine. Polymer hydrogels are in these areas used for example as matrix for controlled release of biologically active agents and cell cultures, may be used for soft tissues augmentation, as temporal substitution of intercellular matrix, etc.

From a physicochemical point of view, polymer hydrogel is a disperse system composed of disperse medium - water and disperse portion that is formed of a three-dimensional macromolecular network. This network forms a continuous structure that permeates through the whole disperse environment. Continuous is thus not only the disperse environment but also the disperse portion. Hydrogels are materials with elastic properties that result from the presence of this three-dimensional macromolecular network.

According to the nature of interactions participating in the forming of polymer network, the gels can be divided into physically crosslinked and chemically crosslinked (covalent gels) [1]. The polymer network of covalent hydrogels may be formed by e.g. polymerization of monomers present in a solution or by crosslinking of originally linear chains of precursor polymers. This may be accomplished e.g. by mutual reaction of suitable functional groups of polymer chains or by using crosslinking agents containing two or more functional groups able to react with an initial polymer. During the crosslinking, the polymer network dimension gradually grows until the whole volume of the disperse system is permeated with the polymer network. The forming of the infinite network is called a gelation point. Growing of the number of knot points and number of involved polymer chains need not, in fact, stop at this point. The mass of the gel portion may continue growing at the expense of disperse environment mass. Further progress of crosslinking reaction then leads to an increase in network density of the hydrogel that may influence e.g. stability, mechanical properties and diffusion parameters of the hydrogel environment.

It is preferred, in a number of medicinal applications that the polymer network formation and hydrogel forming occurs directly in the spot determined for the material application, or *in situ* [2, 3]. Hydrogel formation may be induced by specific conditions (temperature, pH) or by acting of external stimuli (physical - UV irradiation, chemical - reaction initiator, addition of crosslinking agent, etc.).

Suitable method usable for gel preparation *in situ* must meet a number of parameters:
- the process must proceed under physiological conditions
- the kinetics of gel formation and its final properties (network density, mechanical properties, swelling, agent diffusion through the gel matrix) must be perfectly controllable and reproducible
- reactants and products must not be toxic
- in the case of biodegradable hydrogels, the degradation products must not be toxic and must be able to undergo elimination from the organism.

Hyaluronan is a polysaccharide from the group of glycosaminoglycans that consists of units consisting of D-glucuronic acid and N-acetylglucosamine. It is the polysaccharide that is well soluble in aqueous environment, where it forms, depending on molecular mass and concentration, viscous solutions to viscoelastic hydrogels. Hyaluronan is a natural component of intercellular matrix of tissues. By binding to specific topical cellular receptor, the molecule of hyaluronan is able to interact with cells in its environment and regulate their metabolic processes [4]. Hydrogels based on hyaluronan undergo in the organism natural degradation by acting of specific enzymes (hyaluronases), or by acting of reactive oxygen species, resulting in their gradual absorption following their implantation into the organism [5]. For achieving mechanically more resistant materials and due to slowing of their biodegradation a number of hydrogel kinds was developed based on covalently crosslinked hyaluronan. Such hydrogels are often used as materials for viscosupplementation of synovial fluid, soft tissue augmentation, scaffold structures for cultivation and implantation of cells, etc. [6-9].

In the past, various kinds of hyaluronan derivatives were developed that are able to undergo sol-gel transition under physiological conditions in situ [10, 11]. For example, phenolic derivatives of hyaluronan may be used for this purpose. Calabro et al. [12-14] describe in the documents EP1587945B1 and EP1773943B1 the process of preparation of phenolic hyaluronan derivatives with the reaction of carboxylates present in the structure of D-glucuronic acid of hyaluronan, with aminoalkylderivatives of phenol e.g. tyramine. Products of this reaction are hyaluronan amides [15].

Crosslinking of phenolic hyaluronan derivatives may be initiated by an addition of peroxidase (e.g. horseradish peroxidase) and a diluted solution of hydrogen peroxide. Horseradish peroxidase (Horseradish peroxidase, HRP, E.C. 1.11.1.7) is currently widely used as catalyst of organic and biotransformation reactions [16-20]. Hydrogels based on hydroxyphenyl derivatives of hyaluronan may be used as injectable matrix for controlled release of agents or as materials suitable for cultivation and implantation of cells [21]. Wolf et al. describe, in the document CZ303879, a hyaluronan conjugate with tyramine containing aliphatic linker inserted between the chains of polymer and tyramine. The presence of aliphatic linker enables improved efficiency of the crosslinking reaction and gives the network higher elasticity.

Kurisawa *et al.* describe, in the document US8853162, a method of a preparation of a hydrogelcontaining interpenetrating networks based on a hydroxyphenyl derivative of hyaluronan and fibrin. The method uses a reaction catalyzed with horseradish peroxidase for the crosslinking of hydroxyphenyl derivative of hyaluronan. Fibrin network forms from fibrinogen by acting of thrombin. This approach provides hydrogels that are more resistant to biodegradation processes in comparison with hydrogel based on fibrin alone. Hydrogels formed by combination of fibrin network and covalently crosslinked hyaluronan derivatives are used as matrices for cell culture and in future may be used as a part of healing preparations for tissue engineering [22, 23]. A disadvantage of this solution is the use of blood derivatives - thrombin and fibrinogen - obtained by homolog blood processing. This process is costly and at the same time does not eliminate the danger of infectious diseases transmission.

Hydrogel crosslinking is mediated by reaction catalyzed with horseradish peroxidase that results in oxidation of hydroxyphenyl cores of tyramine resulting in dimers, or oligomers thereof. Hydrogen peroxide is a source of hydroxyl radicals for reactions catalyzed by horseradish peroxidase and serves thus as crosslinking reaction initiator. In the environment of blood plasma or blood, the hydrogen peroxide undergoes degradation through interactions with other enzymes (e.g. catalase, myeloperoxidase) [24, 25], or with hematin present in hemoglobin [26]. These are reactions that compete horseradish peroxidase mediated crosslinking reaction, which leads to a decrease in efficiency of hydrogel crosslinking. This phenomenon manifests itself in the case of blood plasma use as longer time of gelation and lower elastic module of formed hydrogel. In the case of hydrogel preparation with the use of full blood may this phenomenon result in the failure of the process of hydrogel formation.

Blood and transfusion preparations made of blood are in the field of regenerative medicine used as source of a number of biologically active agents that participate in tissue defects healing, regulation of inflammation reaction, differentiation of progenitor cells etc. [27, 28].

The possibility of using the combination of *in situ* crosslinking hydrogel and blood as material for support of tissue defects healing is from clinical point of view advantageous because it does not demand either preparation of transfusion preparation or blood derivative production. The possibility of using autologous blood in the same time eliminates the risk of infectious diseases transmission. Combination of blood and *in situ* gelling hydrogel for support of tissue defects healing is described in e.g. EP1294414 [29] that uses a mixture of blood and chitosan-glycerol phosphate for fillings of tissue defects. The solutions of chitosan-glycerol phosphate are known for their ability to undergo the transition of sol-gel by increasing the solution temperature to 37 °C [30, 31]. The use of this system leads to an accelerated production of blood clot and to acceleration of defect closure. Although this process is faster than the natural forming of blood clot, in clinical practice it demands temporal interruption of the intervention, that lasts typically about 15 minutes [32], and that is necessary for the gel to reach suitable mechanical properties [29].

Lee *at al.* describe the use of a hydroxyphenyl derivative of gelatin for a preparation of hydrogels with content of blood plasma fraction reaches in blood platelets that serves as a scaffold for culture of rabbit chondrocytes. Implantation of this scaffold into the model osteochondral defects lead to acceleration of healing of the damaged cartilage. The described system, however, was not used for preparation of hydrogels with blood content.

### Summary of the invention

The goal of the present invention is to develop a means (a set or a kit) that enables the effective preparation of the hydrogel.

Means for use for the hydrogel preparation the principle thereof comprises two separate solutions A and B, where solution A contains enzyme horseradish peroxidase and solution B contains hydrogen peroxide, whereas at least one solution A or B contains calcium ions in the form of pharmaceutically acceptable salt and further solution A and/or B contains hydroxyphenyl derivative of hyaluronan of general formula I 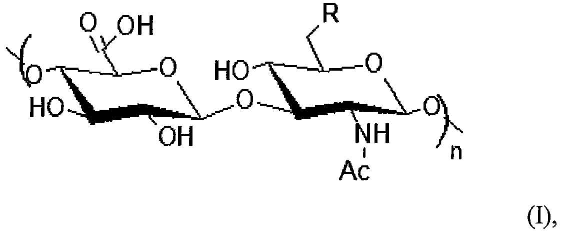 where n is in the range 2 to 7500 and R is OH or substituent NHR₂CONHR₁ArOH of general formula II, 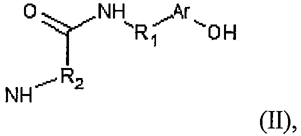 where Ar is phenylene and R₁ ethylene or Ar is indolylene and R₁ is ethylene or Ar is hydroxyphenyl and R₁ is carboxyethylene and R₂ is alkylene of 3 to 7 carbon atoms.

In the means according to one embodiment of the present invention, solution A contains horseradish peroxidase of activity 0.5 to 2.25 U/mL, preferably 1.8 to 2.2 U/mL, more preferably 1.0 to 1.3 U/mL and solution B contains hydrogen peroxide at 2 to 10 mmol/L, preferably 3 to 7 mmol/L, more preferably 4 to 5 mmol/L, whereas at least one solution A or B contains calcium ions at concentration 0.05 to 0.55 mol/L, preferably 0.25 to 0.55 mol/L, more preferably 0.25 to 0.45 mol/L, the most preferably 0.25 to 0.32 mol/L and hydroxyphenyl derivative of hyaluronan of the general formula I at the concentration 10 to 125 mg/mL, preferably 10 to 35 mg/mL, more preferably 20 to 26 mg/mL.

Means according to the present invention comprises two aqueous solutions A and B, one of which contains horseradish peroxidase (solution A) and the other hydrogen peroxide (solution B), whereas at least one of the solutions contains hydroxyphenyl derivative of hyaluronan of general formula I, and at the same time at least one of the solutions contains calcium ions in the form of pharmaceutically acceptable salts and in water well soluble salts. Pharmaceutically acceptable calcium salts are selected from the group containing calcium chloride, calcium lactate, calcium acetate, calcium gluconate, calcium hydrogen carbonate or monocalcium phosphate, preferably calcium chloride, calcium hydrogen carbonate, calcium lactate, calcium acetate and monocalcium phosphate.

Hydroxyphenyl derivative of hylauronan of general formula I may be prepared following the protocols in CZ303879.

Polysaccharides including hyaluronan and derivatives prepared thereof belong among polymers made of mixtures of macromolecules of various sizes and form ununiform (polydisperse) systems. Molar mass of such polymers may be expressed as number average molar mass (Mn) or mass average molar mass (Mw). The ratio of these two types of average molar masses of polymer chains (Mw/Mn) expresses the measure of ununiformity (polydisperisty) of polymer sample and is referred to as polydispersity index (PI). According to another embodiment of the present invention is Mw of hydroxyphenyl derivative of hyaluronan of general formula I in the range 4 × 10⁴ to 3 × 10⁶ g/mol, preferably 1 × 10⁵ to 1 × 10⁶ g/mol, more preferably 2 × 10⁴ to 4 × 10⁵ g/mol. PI is in the range 1 to 3.

According to another embodiment of the present invention is the degree of substitution (DS) of hydroxyphenyl derivative of hyaluronan of general formula I in the range 1 to 10%, preferably 2 to 5%, more preferably 2 to 4%.

Solutions A, B or the precursor solution A are aqueous solutions, preferably 0.9% aqueous solution of sodium chloride, i.e. isotonic solutions, i.e. with the same osmolarity (308 mOsmol) as blood plasma.

Solution A of the described means is used in a preparation of the precursor solution of hydrogel, where solution A of the means is mixed with blood or the transfusion preparation with a content of fibrinogen resulting in the formation of pA. Mixing solutions pA and B according to the present invention in volume ratio 1 : 1 results in formation of gel-forming mixture that after crosslinking of hydroxyphenyl derivative of hyaluronan gives hydrogel containing blood or the transfusion preparation with content of fibrin. The means and the method of preparation of hydrogels described in this document enables the preparation of hydrogels, in which blood or the transfusion preparation with the content of fibrin makes 1 to 30 vol. % of the total volume of the final hydrogel.

According to another embodiment of the present invention the means comprises solutions A and B, where the solution A comprises:
- hydroxyphenyl derivative of hyaluronan of the general formula I of the mass average molar mass in the range 4 × 10⁴ g/mol to 3 × 10⁶ g/mol (and with the polydispersity index 1 - 3), in which the DS is in the range 1 to 10%, whereas it is in the concentration 0 to 125 mg/mL, preferably 10 to 125 ml/mL
- horseradish peroxidase of activity 0.5 to 2.25 U/mL
- calcium ions in the concentration 0.05 to 0.55 mol/L and solution B comprises:
- hydroxyphenyl derivative of hyaluronan of the general formula I of the mass average molar mass in the range 4 × 10⁴ g/mol to 3 × 10⁶ g/mol (and with polydispersity index 1 - 3), in which the DS is in the range 1 to 10%, whereas the concentration is 10 to 50 mg/mL
- hydrogen peroxide in the concentration range 2 to 10 mmol/L.

According to another preferred embodiment of the present invention the means comprises solutions A and B, where the solution A comprises:
- hydroxyphenyl derivative of hyaluronan of the general formula I of mass average molar mass in the range 1 × 10⁵ g/mol to 1 × 10⁶ g/mol (and with polydispersity index 1-3), in which DS is in the range 2 to 5%, whereas it is in the concentration 0 to 35 mg/mL, preferably 10 to 35 mg/mL,
- horseradish peroxidase of activity 0.5 to 2.25 U/mL
- calcium ions at the concentration 0.05 to 0.55 mol/L

And the solution B comprises:
- hydroxyphenyl derivative of hyaluronan of the general formula I of the mass average molar mass 1 × 10⁵ g/mol to 1 × 10⁶ g/mol (and with polydispersity index 1 - 3), in which DS is in the range 2 to 5%, whereas it is at the concentration 26 mg/mL
- hydrogen peroxide in the range of concentration 3 to 7 mmol/L.

According to another preferred embodiment of the present invention the means comprises solutions A and B, where the solution A comprises:
- horseradish peroxidase of activity 1.8 to 2.2 U/mL
- calcium ions at the concentration 0.45 to 0.55 mol/L and the solution B comprises:
- hydroxyphenyl derivative of hyaluronan of the general formula I of the mass average molar mass in the range 2 × 10⁵ g/mol to 4 × 10⁵ g/mol (and with polydispersity index 1 - 3), in which the DS is in the range 2 to 4%, whereas it is at the concentration 26 mg/mL
- hydrogen peroxide in the concentration range 4 to 5 mmol/L.

According to another preferred embodiment of the present invention the means comprises solutions A and B, where the solution A comprises:
- hydroxyphenyl derivative of hyaluronan of the general formula I of the mass average molar mass in the range 2 × 10⁵ g/mol to 4 × 10⁵ g/mol (and with polydispersity index 1 - 3), in which the DS is in the range 2 to 4%, whereas it is at the concentration 20 mg/mL
- horseradish peroxidase of activity 1.0 to 1.3 U/mL
- calcium ions of concentration 0.25 to 0.32 mol/L and the solution B comprises:
- hydroxyphenyl derivative of hyaluronan of the general formula I of the mass average molar mass in the range 2 × 10⁵ g/mol to 4 × 10⁵ g/mol (and with polydispersity index 1 - 3), in which the DS is in the range 2 to 4%, whereas it is at the concentration 26 mg/mL
- hydrogen peroxide in the concentration range 4 to 5 mmol/L.

According to yet another preferred embodiment of the present invention the means comprises solutions A and B, where the solution A comprises:
- horseradish peroxidase of activity 0.8 to 2.2 U/mL
- calcium ions at the concentration 0.02 to 0.5 mol/L
- hydroxyphenyl derivative of hyaluronan of the general formula I of the mass average molar mass in the range 9 × 10⁴ g/mol to 4 × 10⁵ g/mol, with polydispersity index 1 to 10% and at the concentration in the range 10 to 50 mg/mL, and the solution B comprises:
- hydrogen peroxide in the concentration range 4 to 5 mmol/L.

Another embodiment of the present invention is the means of the preparation of the hydrogel comprising the covalently crosslinked hydroxyphenyl derivative of hyaluronan that is made by preparation of two separate solutions A and B described above, and then the solution A is mixed with blood or with at least one transfusion preparation with content of fibrinogen resulting in formation of the precursor solution A, that is mixed with the solution B. Solutions A and B of the means according to the present invention are preferably used in the method according to the present invention.

10 to 60 % (v/v) of blood or the transfusion preparation with content of fibrinogen is preferably added to the solution A, more preferably 30 to 60% (v/v). The precursor solution A with the solution B is preferably mixed in volume ratio 1 : 1.

According to a preferred embodiment of the method of the present invention is at first the solution A of the means mixed with blood or the transfusion preparation with fibrinogen content resulting in the precursor solution of hydrogel pA that comprises:
- hydroxyphenyl derivative of hyaluronan of general formula I of mass average molar mass in the range 4 × 10⁴ g/mol to 3 × 10⁶ g/mol (and with polydispersity index 1 - 3) with substitution degree 1 to 10%, at concentration 0 to 50 mg/mL, preferably 10 to 50 mg/mL,
- horseradish peroxidase of activity 0.2 to 0.9 U/mL,
- calcium ions at concentration 0.02 to 0.22 mol/L,
- 10 to 60% (v/v) of blood or transfusion preparation with the content of fibrinogen, that is further mixed in volume ratio 1 : 1 with thesolution B of above means that comprises:
- hydroxyphenyl derivative of hyaluronan of general formula I of mass average molar mass in the range 4 × 10⁴ g/mol (and with polydispersity index 1 - 3), in which is DS in the range 1 to 10%, whereas it is at the concentration 10 to 50 mg/mL,
- hydrogen peroxide in the range of concentration 2 to 10 mmol/L.

Further is also described a preferred embodiment of the means of the preparation of the hydrogel according to the present invention, where the solution A of the means is at first mixed with blood or the transfusion preparation with content of fibrinogen giving the precursor solution of hydrogel pA that further comprises:
- hydroxyphenyl derivative of hyaluronan of the general formula I of mass average molar mass in the range 1 × 10⁵ g/mol to 1 × 10⁶ g/mol (and with polydispersity index 1 - 3) in which is DS 2 to 5%, whereas it is at the concentration 0 to 14 mg/mL, preferably 10 to 14 mg/mL,
- horseradish peroxidase of activity 0.2 to 0.9 U/mL
- calcium ions at the concentration 0.02 to 0.22 mol/L
- 10 to 60 % (v/v) of blood or the transfusion preparation with fibrinogen content that is further mixed in volume ratio 1 : 1 with the solution B of above described means, that comprises:
- hydroxyfenyl derivative of hyaluronan of general formula I of mass average molar mass in the range 1 × 10⁵ g/mol to 1 × 10⁶ g/mol (and with polydispersity index 1 - 3), in which is DS 2 to 5%, whereas it is at concentration 26 mg/mL
- hydrogen peroxide in the range of concentration 3 to 7 mmol/L.

Further is described another preferred embodiment of the means of the preparation of the hydrogel according to the present invention, where the solution A is at first mixed with the transfusion preparation with content of fibrinogen resulting in the precursor solution of the hydrogel pA that comprises:
- horseradish peroxidase of activity 0.7 to 0.9 U/mL,
- calcium ions at concentration 0.18 to 0.22 mol/L,
- 60% (v/v) of blood plasma or the fraction of blood plasma reaches in blood platelets,
that is further mixed in the volume ratio 1 : 1 with the solution B of the above described means, that comprises
- hydroxyphenyl derivative of hyaluronan of the general formula I at the mass average molar mass in the range 2 × 10⁵ g/mol to 4 × 10⁵ g/mol (and with polydispersity index 1 - 3), in which is DS in the range 2 to 4%, whereas it is at the concentration 26 mg/mL
- hydrogen peroxide in the concentration range 4 to 5 mmol/L.

Further is described yet another preferred embodiment of the preparation of the hydrogel according to the present invention, where the solution A is at first mixed with blood giving the precursor solution of the hydrogel pA that comprises:
- hydroxyphenyl derivative of hyaluronan of the general formula I of the mass average molar mass in the range 2 × 10⁵ g/mol to 4 × 10⁵ g/mol (and with polydispersity index 1 - 3), in which DS is in the range 2 to 4%, whereas it is at the concentration 14 mg/mL
- horseradish peroxidase of activity 0.7 to 0.9 U/mL
- calcium ions at concentration 0.18 to 0.22 mol/L
- 30% (v/v) of blood,

That is further mixed in the volume ratio 1 : 1 with the solution B of the above described means, that comprises:
- hydroxyphenyl derivative of hyaluronan of the general formula (I) of the mass average molar mass in the range 2 × 10⁵ g/mol to 4 × 10⁵ g/mol (and with polydispersity index 1 - 3), in which DS is in the range 2 to 4%, whereas it is at the concentration 26 mg/mL
- hydrogen peroxide in the concentration range 4 to 5 mmol/L.

According to another embodiment of the present invention, hydrogels prepared by the means, as is described above, comprise enzyme horseradish peroxidase of activity 0.1 to 2.5 U/mL, preferably 0.25 to 0.45 U/mL, more preferably 0.35 to 0.45 U/mL, calcium ions at the concentration 0.01 to 0.11 mol/L, preferably 0.09 to 0.11 mol/L, the covalently crosslinked hydroxyphenyl derivative of hyaluronan at the concentration 5 to 50 mg/mL, more preferably 13 to 20 mg/mL and blood or the transfusion preparation with fibrinogen content at the concentration in the range 5 to 30% (v/v) preferably 15 to 30% (v/v). Hydrogels elasticity (G') according to the present invention may fall in the range from 1 Pa to 3 kPa, preferably 1 Pa to 2 kPa.

According to another embodiment of the present invention, the hydrogel described above will be used for manufacturing of preparations in cosmetics, medicine or regenerative medicine.

Preferably, such preparation is chosen from a group of preparations comprising wound dressing, preparation for soft tissues augmentation, preparations for viscosupplementation of synovial fluid, matrix for controlled drug release, fillings of tissue defects, scaffolds for tissue engineering.

As stated above, the preparation according to the present invention enables the preparation of gel-forming mixture (hydrogel) that forms following mixing of hydrogel precursor solution A and solution B. Gel-forming mixture (hydrogel) contains hydroxyphenyl derivative of hyaluronan of the general formula I, blood or the transfusion preparation containing fibrinogen, horseradish peroxidase, hydrogen peroxide and calcium ions in the form of water soluble pharmaceutically acceptable salt. This gel-forming mixture enables forming of hydrogel based on the covalently crosslinked tyramine derivative of hyaluronan with content of blood or the transfusion preparation containing fibrin.

To the hydrogel crosslinking contributes the reaction catalyzed with horseradish peroxidase that leads to oxidation of hydroxyphenyl cores of tyramine resulting in dimer, or oligomer formation. Hydrogen peroxide is source of hydroxyl radicals for reaction catalyzed with horseradish peroxidase and serves as the crosslinking reaction initiator.

Surprisingly, it was found that the addition of sufficient amount of calcium ions into at least one solution A or B, as is stated above, makes the process of gelation more effective and increases the elasticity module of forming hydrogels. This phenomenon has not been observed in analogous hydrogels without content of blood or the transfusion preparation.

It was further found that the presence of calcium ions in the solution of hydroxyphenyl derivative or its mixture with blood or the transfusion preparation with content of fibrinogen itself does not lead, without the initiation of enzymatic crosslinking reaction of hydroxyphenyl derivative of hylauronan, to the formation of hydrogels. It is not only a manifestation of the blood coagulation that may result from the increased calcium concentration or the result from calcium ions interaction with the polyanionic chain of the hydroxyphenyl derivative of hyaluronan. It can't be excluded that all mentioned processes take place simultaneously in the gel-forming mixture of the hydrogel. It is likely, that the formation of the hydrogel occurs by interaction among individual forming polymer networks and their combined size exceeds critical limit and hydrogel forming occurs. Contrary to so far published systems [34], thus described composition of the means enables the preparation of the hydrogel based on the hydroxyphenyl derivative of hyaluronan of the general formula I also in the presence of blood.

In the case of blood use, above described gel-forming mixtures enable forming of the hydrogel at hydrogen peroxide concentrations that don't lead to damage of biological structures and at the same time enable use of horseradish peroxidase of activity that wouldn't lead itself to effective hydrogel forming in the presence of blood or the transfusion preparation with fibrinogen content. This significantly increases the possibility to use the means in the field of tissue engineering, regenerative medicine and further medicinal fields.

Similar phenomenon may be observed also in the case of hydrogels with the content of plasma. Hydrogen peroxide is more stable in plasma and thus the presence of calcium ions is not in this case essential for the hydrogel forming. Nevertheless, in this case too, the presence of calcium ions leads to acceleration of the process of gelation and increases the elastic modules of the prepared hydrogels. This phenomenon may be also explained by the parallel forming of the fibrin network and the network based on the hydroxyphenyl derivative of hyaluronan.

Higher concentration of calcium ions leads to inhibition of proliferation of cultured cells, what would limit the use of the means as material for medicinal purposes. In the means according to the present invention, the amount of calcium ions is optimized so that it is sufficient for support of forming hydrogels of the invention but at the same time enables use of prepared hydrogels of the invention as matrix for 2D and 3D cell culture. The possibility to use a combination of blood or transfusion preparations containing fibrinogen for the preparation of hydrogels based on hydroxyphenyl derivatives of hyaluronan was not so far published.

The term "blood" means blood drawn from a donor, human or animal. It refers to a heterologous donor or preferably autologous donor. It is a material for the preparation of transfusion preparations. The term"transfusion preparation with fibrinogen content" means zje transfusion preparation that is made from blood by usual protocols. The transfusion preparation is selected from a group containing blood plasma, plasma fraction reaches in blood platelets (PRP). It does not refer to blood derivatives, that are industrially manufactured preparations that comprise especially albumin, coagulating factors and immunoglobulins of human origin.

### Brief description of the drawings

Fig. 1 Effect of concentration of calcium ions on gelation rate of samples containing the transfusion preparation - 30 % (v/v) plasma, 13 mg/mL solution HATA, HRP 0.4 U/mL, H₂O₂ 2.3 mM, various concentrations of CaCl₂.2H₂O in hydrogel.
Fig. 2 Effect of concentration of Ca²⁺ (mol/L) on proliferation of 3T3 cells.
Fig. 3 Cytotoxicity of hydrogel extracts containing various concentrations of calcium ions
Fig. 4 Cytotoxicity of hydrogel extracts containing various ratios of blood/blood plasma. Concentration of calcium ions in the gel was equal to 0.1 mol/L. Given % are % (v/v).
Fib. 5 Effect of concentration of Ca²⁺ ions in hydrogels containing 30 % (v/v) ratio of blood plasma.
Fig. 6 Cell adhesion on the surface of hydrogels - staining LIVE/DEAD. Given % are % (v/v).
Fig. 7: 3D culture of cells - staining LIVE/DEAD (arrows - adhered cells). Given % are % (v/v).

### Examples of the embodiments of the invention

DS = degree of substitution = 100 % * molar amount of modified disaccharide units of hyaluronan / molar amount of all disaccharide units of the derivative of hyaluronan. Degree of substitution was determined with ¹H NMR spectroscopy.

Mass average molar mass (Mw) and polydispersity index (PI) were determined using SEC-MALLS.

### Example 1

### Synthesis of tyramine derivative of HA (HA-TA)

### Synthesis of 6-amino-N-[2(4hydroxyphenyl)ethyl]hexanamide

6-[(*terc.* butoxycarbonyl)amino]hexane acid (1.00 g, 4.3 mmol) was dissolved in 50 mL tetrahydrofuran (THF). 1,1'carbodiimidazole (0.70 g, 4.3 mmol) was added to the acid solution. The reaction mixture was heated at 50 °C for sixty minutes. The container was then washed with inert gaz. Tyramine (0.59 g, 4.3 mmol) was added to the reaction mixture. The mixture was further heated for another 2 hours. THF was then removed by distillation under reduced pressure. The residue was dissolved in 50 mL ethylacetate. The solution was washed with 150 mL purified water (divided into three parts). Organic layer was dried over molecular sieve. Ethylacetate was removed by distillation under reduced pressure. The residue was heated for 6 hours at reflux. The solvent was removed by distillation under reduced pressure. The residue was dissolved in 50 mL ethylacetate. The solution was washed with 150 mL of purified water (divided into three parts). Organic layer was dried over molecular sieve. Ethylacetate was removed by distillation under reduced pressure.
m = 0.75 g (70 % theory)

¹H NMR (D₂O, ppm) δ: 1.17 (m, 2 H, γ-CH₂-hexane acid); 1.48(m, 2 H, β-CH₂-hexane acid); 1.58 (m, 2 H, δ-CH₂-hexane acid); 2.17 (t, 2 H, -CH₂-CO-); 2.73 (m, 2 H, -CH₂-Ph); 2.91 (m, 2 H, -CH₂-NH₂); 3.42 (m, 2 H, -CH₂-NH-CO-); 6.83 (d, 2 H, arom); 7.13 (d, 2 H, arom).

¹³C NMR(D₂O, ppm) δ: 24 (γ-C-hexane acid); 26 (δ-C-hexane acid); 33 (β-C-hexane acid); 35 (-C-CO-); 39 (-C-NH₂); 40 (C-Ph); 63 (-C-NH-CO-); 115 (C3 arom); 126 (C1 arom); 130 (C2 arom.); 153 (C4 arom); 176 (-CO-).

### Preparation of aldehyde derivative (HA-CHO)

Hyaluronan (10.00 g, Mw = 2 × 10⁶ g.mol⁻¹) was dissolved in 750 mL 2.5% (w/w) solution Na₂HPO₄.12 H₂O. The solution was cooled to 5 °C. 2.60 g NaBr and 0.05 g 4-acetamido-2,2,6,6-tetramethylpiperidine-1-oxyl was added to resulting solution. After thorough homogenization of the solution, 3 mL of solution NaClO (10-15 % available Cl₂) were added to reaction mixture. Reaction proceeded under continuous stirring for 15 min. Reaction was quenched by addition of 100 mL 40% solution of propan-2-ol. Product was purified by ultrafiltration and isolated by precipitation with propan-2-ol.

IR (KBr): 3417, 2886, 2152, 1659, 1620, 1550, 1412, 1378, 1323, 1236, 1204, 1154, 1078, 1038, 945, 893 cm⁻¹.

¹H NMR (D₂O) δ: 2.01 (s, 3 H, CH₃-), 3.37 - 3.93 (m, skeleton of hyaluronan), 4.46 (s, 1H, anomer), 4.54 (s, 1H anomer., -O-CH(OH)-), 5.27 (geminal glykol -**CH-**(OH)₂).

### a) Preparation of tyramine derivative of HA with C₆ spacer (Mw ≈ 5 × 10⁴ g.mol⁻¹, DS ≈ 6 %)

Aldehyde derivative of HA (≈6 × 10⁴ g.mol⁻¹, DS = 9 %) (5.00 g) was dissolved in 500 mL demineralized water. pH was adjusted with acetic acid to 3. 6-amino-*N*-[2-(4-hydroxyphenyl)ethyl]hexanamide (intermediate (I)) (1.25 g, 5 mmol) was added to solution of HA-CHO. The mixture was stirred for 2 hours at room temperature. Complex picoline-borate (0.270 g, 2.5 mmol) was then added to the reaction mixture. The mixture was stirred for another 12 hours at room temperature. Product was purified by ultrafiltration and isolated from retentate by precipitation with propan-2-ol. Humidity and residual propan-2-ol were removed from precipitate by drying in hot-air drier (40 °C, 3 days).

IR (KBr):: 3425, 2893, 2148, 1660, 1620, 1549, 1412, 1378, 1323, 1236, 1204, 1154, 1078, 1038, 945, 893 cm⁻¹.

¹H NMR (D₂O) δ: 1.25 (t, 2 H, γ -CH₂- aminohexane acid), 1.48 (m, 2 H, δ -CH₂-aminohexane acid) 1.51 (m, 2 H, β -CH₂- aminohexane acid), 2.01 (s, 3 H, CH₃-), 2.65 (m, 2H, Ph-CH₂-), 2.73 (m, 2H, ε-CH₂- aminohexane acid), 3.37 - 3.93 (m, skelet of hyaluronan), 4.46 (s, 1H, anomer), 4.54 (s, 1H anomer., -O-CH(OH)-), 6.59 (d, 2H, arom.), 7.01 (d, 2H. arom).
SEC MALLS: Mw = 5,4 × 10⁴ g.mol⁻¹; PI = 1,61
DS (¹H NMR): 6.2 %

### b) Preparation of tyramine derivative of HA with C₆ spacer (Mw ≈ 3 × 10⁵ g.mol⁻¹, DS ≈ 2%)

Aldehyde derivative of HA (Mw ~ 3 × 10⁵ g.mol⁻¹, DS ~ 9%) (5.00 g) was dissolved in 500 mL demineralized water. pH was adjusted with acetic acid to 3. 6-amino-*N*-[2-(4-hydroxyphenyl)ethyl]hexanamid (intermediate (I)) (0.625 g, 2.5 mmol) was added to the solution of HA-CHO. The mixture was stirred for 2 hours at room temperature. Complex picoline-borate (0.270 g, 2.5 mmol) was then added to the reaction mixture. The mixture was stirred for another 12 hours at room temperature. The product was purified by ultrafiltration and isolated from retentate by precipitation with propan-2-ol. Humidity and residual propan-2-ol were removed from precipitate by drying in hot-air drier (40 °C, 3 days).

IR (KBr):: 3425, 2893, 2148, 1660, 1620, 1549, 1412, 1378, 1323, 1236, 1204, 1154, 1078, 1038, 945, 893 cm⁻¹.

¹H NMR (D₂O) δ: 1.25 (t, 2 H, γ -CH₂- aminohexane acid), 1.48 (m, 2 H, δ -CH₂-aminohexane acid) 1.51 (m, 2 H, β -CH₂- aminohexane acid), 2.01 (s, 3 H, CH₃-), 2.65 (m, 2H, Ph-CH₂-), 2.73 (m, 2H, ε-CH₂- aminohexane acid), 3.37 - 3.93 (m, skelet of hyaluronan), 4.46 (s, 1H, anomer), 4.54 (s, 1H anomer., -O-CH(OH)-), 6.59 (d, 2H, arom.), 7.01 (d, 2H. arom).
SEC MALLS: Mw = 2.81 × 10⁵ g.mol⁻¹; PI = 1.49
DS (¹H NMR): 2.2 %

### c) Preparation of tyramine derivative of HA with C₆ spacerem (Mw ≈ 1.5 × 10⁶ g.mol⁻¹, DS = 1 %)

Aldehyde derivative of HA (≈1.5 × 10⁶ g.mol⁻¹, DS = 4 %) (5,00 g) was dissolved in 500 mL demineralized water. pH was adjusted with acetic acid to 3. 6-amino-*N*-[2-(4-hydroxyfenyl)ethyl]hexanamid (meziprodukt (I)) (0.625 g, 2.5 mmol) was added to solution of HA-CHO. The mixture was stirred for 2 hours at room temperature. Picoline-borate complex (0.270 g, 2.5 mmol) was then added to reaction mixture. The mixture was stirred for another 12 hours at room temperature. The product was purified by ultrafiltration and isolated from retentate by precipitation with propan-2-ol. Humidity and residual propan-2-ol were removed from precipitate by drying in hot-air drier (40 °C, 3 days).

IR (KBr):: 3425, 2893, 2148, 1660, 1620, 1549, 1412, 1378, 1323, 1236, 1204, 1154, 1078, 1038, 945, 893 cm⁻¹.

¹H NMR (D₂O) δ: 1.25 (t, 2 H, γ -CH₂- aminohexane acid), 1.48 (m, 2 H, δ -CH₂-aminohexane acid) 1.51 (m, 2 H, β -CH₂- aminohexane acid), 2.01 (s, 3 H, CH₃-), 2.65 (m, 2H, Ph-CH₂-), 2.73 (m, 2H, ε-CH₂- aminohexane acid), 3.37 - 3.93 (m, skelet of hyaluronan), 4.46 (s, 1H, anomer), 4.54 (s, 1H anomer., -O-CH(OH)-), 6.59 (d, 2H, arom.), 7.01 (d, 2H. arom).
SEC MALLS: Mw = 1.45 × 10⁶ g.mol⁻¹; PI = 1.65
DS (¹H NMR): 1.1 %

### d) Preparation of tyramine derivative of HA with C₆ spacerem (Mw ≈ 1.0 × 10⁵ g.mol⁻¹, DS = 8.5 %)

Aldehyde derivative of HA (≈1.0 × 10⁵ g.mol⁻¹, DS = 10 %) (5,00 g) was dissolved in 500 mL demineralized water. pH was adjusted with acetic acid to 3. 6-amino-*N*-[2-(4-hydroxyfenyl)ethyl]hexanamid (intermediate (I)) (0.625 g, 2.5 mmol) was added to solution of HA-CHO. The mixture was stirred for 2 hours at room temperature. Complex picoline-borate (0.270 g, 2.5 mmol) was then added to the mixture. The mixture was stirred for another 12 hours at room temperature. The product was purified by ultrafiltration and isolated from retentate by precipitation with propan-2-ol. Humidity and residual propan-2-ol were removed from precipitate by drying in hot-air drier (40 °C, 3 days).

IR (KBr):: 3425, 2893, 2148, 1660, 1620, 1549, 1412, 1378, 1323, 1236, 1204, 1154, 1078, 1038, 945, 893 cm⁻¹.

¹H NMR (D₂O) δ: 1.25 (t, 2 H, γ -CH₂- aminohexane acid), 1,48 (m, 2 H, δ -CH₂-aminohexane acid) 1,51 (m, 2 H, β -CH₂- aminohexane acid), 2.01 (s, 3 H, CH₃-), 2.65 (m, 2H, Ph-CH₂-), 2.73 (m, 2H, ε-CH₂- aminohexane acid), 3.37 - 3.93 (m, skelet of hyaluronan), 4.46 (s, 1H, anomer), 4.54 (s, 1H anomer., -O-CH(OH)-), 6.59 (d, 2H, arom.), 7.01 (d, 2H. arom).
SEC MALLS: Mw = 9.5 × 10⁴ g.mol⁻¹; PI = 1.55
DS (¹H NMR): 8.6 %

### e) Preparation of tyramine derivative of HA with C₆ spacerem (Mw ≈ 2.5 × 10⁶ g.mol⁻¹, DS = 1 %)

Aldehyde derivative of HA (~2.5 × 10⁶ g.mol⁻¹, DS = 4 %) (5,00 g) was dissolved in 500 mL demineralized water. pH was adjusted with acetic acid to 3. 6-ainino-*N*-[2-(4-hydroxyfenyl)ethyl]hexanamide (intermediate (I)) (0.625 g, 2.5 mmol) was added to solution of HA-CHO. The mixture was stirred for 2 hours at room temperature. Complex picoline-borate (0.270 g, 2.5 mmol) was then added to the mixture. The mixture was stirred for another 12 hours at room temperature. The product was purified by ultrafiltration and isolated from retentate by precipitation with propan-2-ol. Humidity and residual propan-2-ol were removed from precipitate by drying in hot-air drier (40 °C, 3 days).

IR (KBr):: 3425, 2893, 2148, 1660, 1620, 1549, 1412, 1378, 1323, 1236, 1204, 1154, 1078, 1038, 945, 893 cm⁻¹.

¹H NMR (D₂O) δ: 1.25 (t, 2 H, γ -CH₂- aminohexane acid), 1.48 (m, 2 H, δ -CH₂-aminohexane acid) 1.51 (m, 2 H, β -CH₂- aminohexane acid) 2.01 (s, 3 H, CH₃-), 2.65 (m, 2H, Ph-CH₂-), 2.73 (m, 2H, ε-CH₂- aminohexane acid), 3.37 - 3.93 (m, skelet of hyaluronan), 4.46 (s, 1H, anomer), 4.54 (s, 1H anomer, -O-CH(OH)-), 6,59 (d, 2H, arom.), 7,01 (d, 2H. arom).
SEC MALLS: Mw = 2.5 × 10⁶ g.mol⁻¹; PI = 1.65
DS (¹H NMR): 1 %

### Example 2

### The influence of the presence of calcium ions on the formation of the hydrogel in the presence of blood

The rate of gelation was measured with rotational rheometer AR-G2 (TA Instrument) using geometry board-board (40 mm) and space size 400 µm. 525 µL sample was applied on bottom static board. Pre-shear 2000 L/s per sec was chosen for sample homogenization. Time sweep mode was chosen for determination of gelation rate at frequency 1 Hz and displacement 0.001 rad at 37 °C. Elastic and viscose module was determined from the dependency of elastic G' on viscose G" module on time t. Tyramine derivative of hyaluronan synthetized following procedure according to example 1 (Mw = 2.81 × 10⁵ g.mol⁻¹, DS 2.2 %) was used for sample preparation.

Elastic G" and viscose G" module of individual samples read at 300 s are presented in Table 1.

**Table 1. Composition of tested gel-forming mixtures**

| | **Concentration HA-TA (mg/mL)** | **Concentration CaCl₂H₂O (mol/L)** | **Activity HRP (U/mL)** | **Concentration H₂O₂ (mmol/L)** | **Proportion of blood in sample (%)** | **G' (Pa)** | **G" (Pa)** |
|---|---|---|---|---|---|---|---|
| 1 | 20 | - | - | - | 15 | 0.17 | 1.23 |
| 2 | 20 | 0.1 | - | - | 15 | 0.34 | 1.06 |
| 3 | 20 | 0.1 | 0.4 | - | 15 | 0.20 | 1.15 |
| 4 | 20 | - | 0.4 | - | 15 | 0.24 | 1.27 |
| 5 | 20 | - | - | 2.3 | 15 | 0.07 | 0.92 |
| 6 | 20 | 0.1 | 0.4 | 2.3 | 15 | 21.80 | 11.50 |
| 7 | 20 | | 0.4 | 2.3 | 15 | 0.52 | 1.99 |

From given data it is apparent that gelation occurs only at the combination of hydroxyphenyl derivative of hyaluronan with horseradish peroxidase, hydrogen peroxide and calcium ions with blood (sample 6) - elastic module G' is higher than viscose module G". In the other samples (samples 1 to 5 and 7) is elastic module lower than the viscose module and the gelation of given mixtures does not occur even after 24 hours.

### Example 3

### Influence of calcium ions on the efficiency of the gelation in the presence of blood and transfusion preparations

Viscoelastic properties of hydrogels were determined with rotational rheometer AR-G2 (TA Instruments) using method strain sweep at constant value of frequency 1 Hz and displacement in the range 10⁻³ to 2 radians. Geometry with rougher surface (cross-hatched) was used in order to avoid slipping of prepared hydrogels during measurement. Hydrogels of diameter 17.5 mm were used for the measurement. Hydrogels were scored after 60 minutes of preparation. Tyramine derivative of hyaluronan synthetized following procedure according to Example 1 (Mw = 2.81 × 10⁵ g.mol⁻¹, DS 2.2 %) was used for the solution preparation.

Viscoelastic properties (elastic G" and viscose G" module) of hydrogels prepared without transfusion preparations are shown in Table 2. The table also demonstrates the difference in the elastic module in hydrogels prepared in the absence and the presence of calcium ions.

No increase in the elastic module depending on the calcium ions presence was observed in hydrogels prepared in the absence of blood or transfusion preparations. On the contrary, hydrogels with the content of blood plasma or blood plasma reaches in blood platelets (PRP) prepared in the presence of calcium ions showed elevated values of the elastic module in comparison with analogous gels prepared in the absence of calcium. Precursor solutions with blood content but without calcium were not able form hydrogel at all.

**Table 2. Composition of tested gel-forming mixtures and properties of hydrogels formed thereof**

| | **Conc. HA-TA (mg/mL)** | **Conc. CaCl₂H₂O (mol/L)** | **Activity HRP (U/mL)** | **Conc. H₂O₂ (mmol/L)** | **(v/v)** | **G' (Pa)** | **SD** | **G" (Pa)** | **SD** |
|---|---|---|---|---|---|---|---|---|---|
| 1 | 13 | 0.1 | 0.4 | 2.3 mM | Without blood | 1028.9 | 48.8 | 2.08 | 0.47 |
| | | 0 | | | | 1052.6 | 34.4 | 4.17 | 1.53 |
| 2 | | 0.1 | | | 30 % plasma | 578.3 | 27.2 | 2.35 | 0.18 |
| | | 0 | | | | 433.7 | 8.5 | 2.07 | 0.05 |
| 3 | | 0.1 | | | 30 % PRP | 548.2 | 65.5 | 2.46 | 0.27 |
| | | 0 | | | | 360.8 | 11.7 | 2.94 | 0.15 |
| 4 | 20 | 0.1 | | | Without blood | 1764.0 | 151.8 | 1.82 | 1.06 |
| | | 0 | | | | 1664.8 | 91.6 | 1.86 | 1.01 |
| 5 | | 0.1 | | | 15 % Blood | 59.0 | 1.9 | 20.73 | 1.85 |
| | | 0 | | | | No gelling | | | |
| 6 | 30 | 0.1 | | | 30 % Blood | 1.7 | 0.3 | 0.41 | 0.06 |
| | | 0 | | | | No gelling | | | |

### Example 4

### Influence of calcium ions on the rate of the hydrogel forming in the presence of blood plasma

Gelation rate was determined using method described in Example 2.

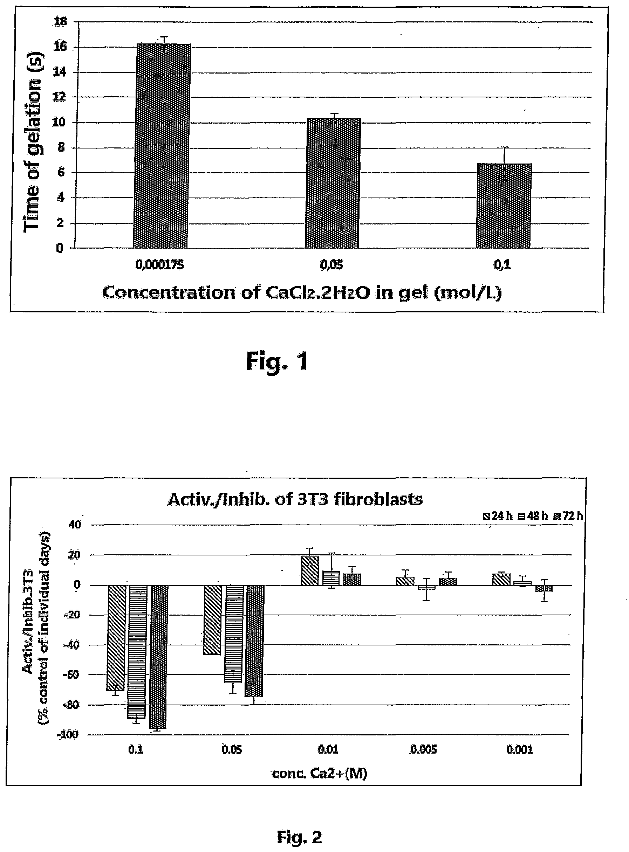

Influence of calcium ions on the rate of gelation of samples containing the transfusion preparation - 30 % (v/v) plasma is shown in Fig.1. The time of gelation decreases with increasing calcium ions concentration.

### Example 5

### Influence of calcium ions on cell proliferation

Fig. 2 shows the viability of cells (fibroblast cell line 3T3) cultured in the presence of calcium ions (0.1 - 0.001 mol/L). The cells were seeded on 24-well culture panel at a density of 15 000 cell/well. The medium was removed the next day; and fresh medium contained calcium ions at tested concentrations. Cell proliferation was determined at intervals 24, 48 and 72 h with assay for determination of ATP using CellTiter-Glo assay. DEMI water in 10% volume of medium was used as the control.

Norm ISO 10993-5-2009 states the limit for cytotoxicity as reduction of viability of influenced cell by more than 30% in comparison with control. Such reduction in cell viability below this limit occurs at concentrations 0.1 mol/L to 0.05 mol/L Ca²⁺. The presence of calcium ions in concentration ≤ 0.01/L does not negatively influence cell viability.

### EXAMPLE 6

### Cytotoxicity of extracts from hydrogels containing various calcium ions concentration

Hydrogels were prepared from gel-forming mixture that came from mixing precursor solution pA and solution B in the volume ratio 1:1 (for composition of solutions and forming hydrogels see Table 3). Calcium chloride was used as source of calcium ions. Tyramine derivative of hyaluronan synthetized according to procedure of Example 1 (Mw = 2.8 × 10⁵ g.mol⁻¹, DS 2.2 %) was used for the preparation of solutions.

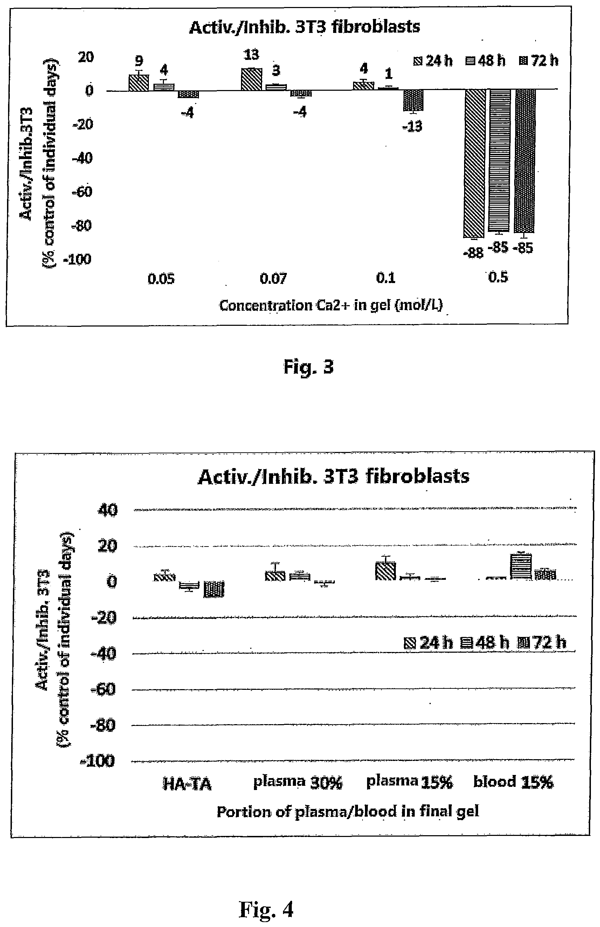

Sterile prepared hydrogels were transferred into a plastic test tube and covered with a culture medium in ratio 9 : 1 (9 mL medium! 1 g gel). Extraction was performed for 72 h at 37 °C. Cells 3T3 were seeded into 24-well culture panel at a density of 15 000 cells/well. The medium was replaced the next day with test extract. Cell proliferation was recorded at the intervals 24, 48 and 72 h by means of ATP determination with CellTiter-Glo assay. Culture medium was used as control. Fig. 3 describes cell viability in the presence of extracts of the hydrogel containing various calcium ion concentrations. The limit value of calcium ions concentration in the hydrogel for cell viability was 0.1 mol/L. Exceeding this limit resulted in hydrogel extracts cytotoxicity.

**Table 3: Concentration of individual components in the precursor solution pA and solution B and the gel-forming mixture produced by mixing the solution pA and the solution B**

| Precursor solution pA | | Solution pB | | Gel-forming mixture (hydrogel) | | | |
|---|---|---|---|---|---|---|---|
| HRP (U/mL) | Ca²⁺ (mol/L) | HA-TA (mg/mL) | H₂O₂ (mmol/L) | HRP (U/mL) | Ca²⁺ (mol/L) | HA-TA (mg/mL) | H₂O₂ (mmol/L) |
| 0.8 | 0.1 | 26 | 4.6 | 0.4 | 0.05 | 13 | 2.3 |
| | 0.14 | | | | 0.07 | | |
| | 0.2 | | | | 0.1 | | |
| | 1 | | | | 0.5 | | |

### Example 7

### Cytotoxicity of extracts of hydrogel containing blood or blood plasma

Preceding the preparation of the hydrogel, solution A was mixed with appropriate blood or blood plasma amount producing precursor solution pA. Hydrogels were produced from gel-forming mixture that resulted from mixing precursor solution pA with addition of blood or blood plasma and solution B in volume ratio 1 : 1 (for composition of solutions and produced hydrogels see table 4). Calcium chloride was used as source of calcium ions. Tyramine derivative of hyaluronan synthetized following procedure according to Example 1 (Mw = 2.81 × 10⁵ g.mol⁻¹, DS 2.2 %) was used for solution preparation. Sterile prepared hydrogels were transferred into the plastic test tube and covered with the culture medium in ratio 9 : 1 (9 mL medium/1 g gel). Extraction proceeded for 72 h at 37 °C. 3T3 cells were seeded into 24-well culture panel at a density of 15 000 cells/well. The medium was replaced with tested extract the next day. Cell proliferation was monitored at intervals 24, 48 and 72 h by means of determination of ATP using CellTiter-Glo assay. Culture medium was used as control. The results presented in Fig. 4 show that extracts of hydrogels with 15 - 30 % (v/v) proportion of blood or blood plasma and with 0.1 mol/L content of calcium ions do not have negative influence on cell viability.

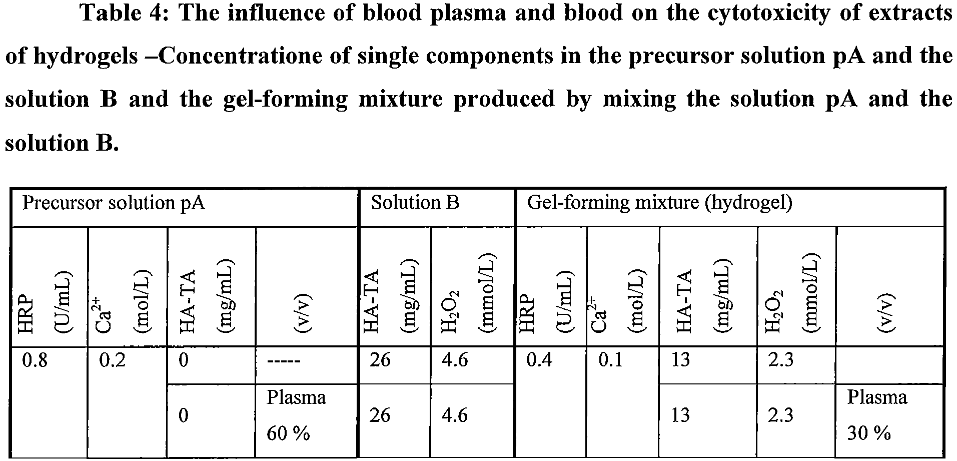

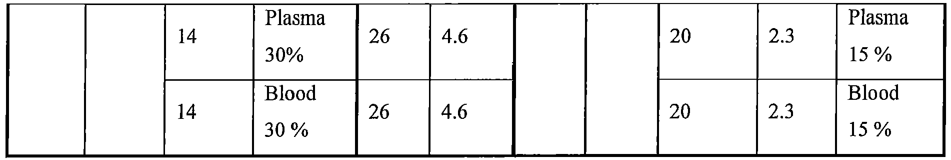

### Example 8

### Cytotoxicity of extracts of the hydrogel containing the portion of blood plasma and various concentration of calcium ions

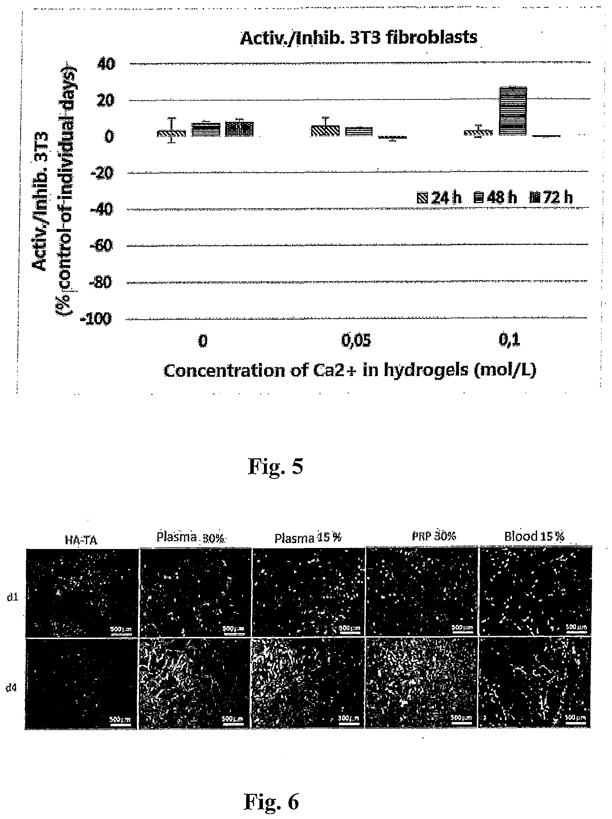

Further, the influence of calcium ions concentration present in hydrogel with the content of blood plasma on the cell viability was studied. For the evaluation of this parameter, the extracts of hydrogels were again prepared. The solution A was mixed with the appropriate amount of blood or blood plasma giving the precursor solution pA before the hydrogel preparation. Hydrogels were prepared from the gel-forming mixture that was produced by the mixing precursor solution pA and the solution B in the volume ratio 1:1 (for the composition of solutions and produced hydrogels see Table 5). Calcium chloride was used as source of calcium ions. Tyramine derivative of hyaluronan synthetized following the procedure according to Example 1 (Mw = 2.81 × 10⁵ g.mol⁻¹, DS 2.2%) was used for solutions preparation. Sterile prepared hydrogels were transferred into a plastic test tube and covered with the culture medium in ratio 9:1 (9 mL medium/1 g gel). Extraction proceeded for 72 h at 37 °C. 3T3 cells were seeded into 24-well culture panel at a density of 15 000 cells/well. The medium was replaced for the tested extract the next day. Cell proliferation was monitored at intervals 24, 48 and 72 h by means of determination of ATP using CellTiter-Glo assay. Culture medium was used as control. The results presented in Fig. 5 show that extracts prepared from hydrogels containing 30% (v/v) portion of cell plasma and calcium ions in concentration range 0 to 0.1 mol/L do not induce cytotoxicity.

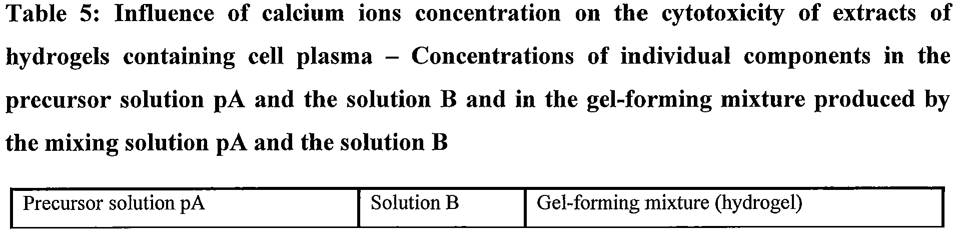

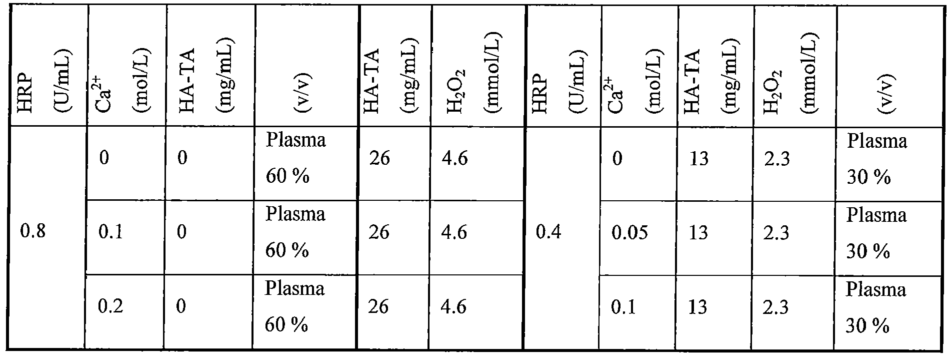

### Example 9

### Test of cell adhesion to hydrogel layers

The solution A was mixed with appropriate amount of blood, blood plasma or PRP giving the precursor solution pA. Hydrogels were produced from the gel-forming mixture that was produced by the mixing precursor solution pA with addition of blood or the transfusion preparation and the solution B in the volume ratio 1:1 (for the composition of solutions and produced hydrogels see Table 6). Calcium chloride was used as a source of calcium ions. Tyramine derivative of hyaluronan synthetized following the procedure according to Example 1 (Mw = 2.81 × 10⁵ g.mol⁻¹, DS 2.2 %) was used for the preparation of the solutions. Tested hydrogels were layered upon 24-well culture panels. Stem cells were then seeded upon the surface of hydrogel layer at a density of 13 000 cells/well. Cell growth was monitored for 4 days using LIVE/DEAD staining.

Stem cell adhesion on the surface of the material manifests itself by typical changes in cell morphology where the cells adopt elongated shape. Fig. 6 documents that the cells are not able to adhere to the hydrogel without the addition of blood or the transfusion preparation. In this case the cells keep, even after 4 days of culture, spherical shape and do not manifest marks of interaction with the surface of the gel. Likewise, their proliferation is suppressed. Hydrogels with the content of blood or transfusion preparations with the fibrinogen content (blood plasma, PRP), on the contrary, support adhesion and proliferation of the cells. Cells adopt on their surface typical elongated shape and their count increases with the time of culture. The experiment also showed that the presence of calcium in structure of hydrogel is not an obstacle for culture upon the hydrogel surface.

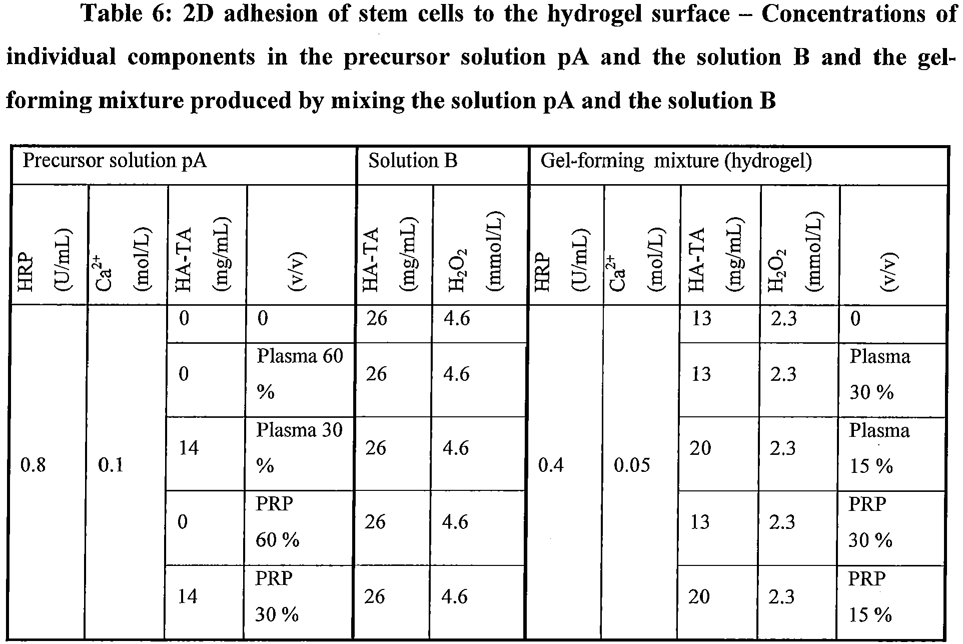

### Example 10

### 3D cell culture

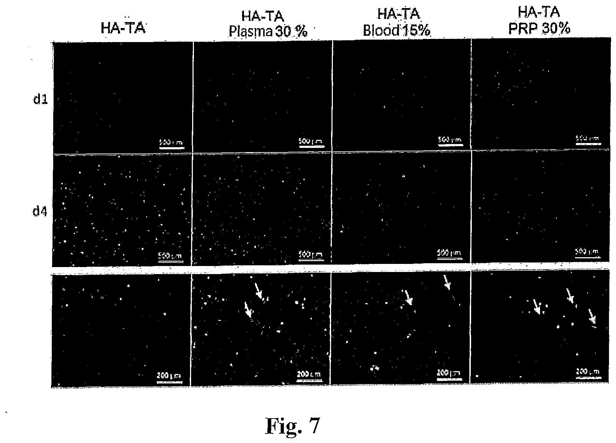

Cells of 3T3 mouse fibroblast cell line were used for encapsulation. Cell pellet was suspended in the solution A before the hydrogel preparation. The solution A was then mixed with the appropriate amount of blood, blood plasma or PRP producing the precursor solution pA. Hydrogels were then prepared by the mixing precursor solution A and the solution B in volume ratio 1:1 (for composition of solutions and produced hydrogels see Table 7). Calcium chloride was used as a source of calcium ions. Tyramine derivative of hyaluronan synthetized following the procedure according to Example 1 (Mw = 2.81 × 10⁵ g.mol⁻¹, DS 2.2 %) was used for the preparation of the solutions. Sterile prepared hydrogels were transferred into the culture panel and covered with the culture medium where they were incubated for 4 days at 37 °C, 5% CO₂. Cell growth was monitored for 4 days using LIVE/DEAD staining. The results showed in Fig. 7 document that the system enables encapsulation of viable cells into hydrogels and their subsequent 3D culture. Cells in hydrogels containing transfusion preparation are able of adhesion.

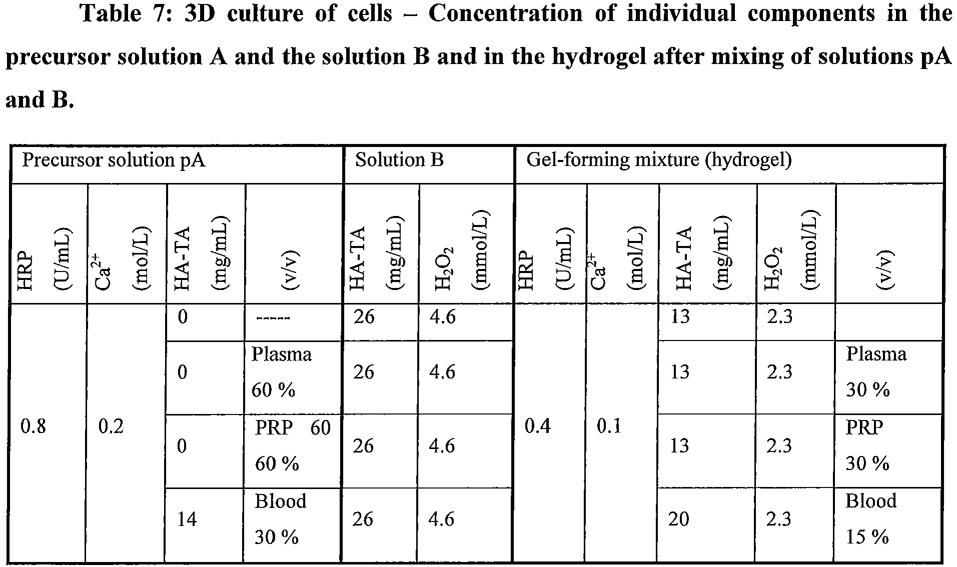

### Example 11

### Preparation of hydrogels with blood content (5% v/v)

Derivative HA-TA (Mw = 5.4 × 10⁴ g.mol⁻¹, DS 6.2 %) was used for preparation of solutions of the means for hydrogel preparation. The precursor hydrogel solution pA was prepared from solution A (1.8 ml) of the means for hydrogel preparation having composition:

| | |
|---|---|
| HRP | 1 U/ml |
| CaCl₂.2H₂O | 0.223 mol/L |
| HA-TA | 55.556 mg/mL, |

by addition of 0.2 ml of blood. Further, 2 ml of solution B of the means were used for hydrogel preparation. Table 8 shows composition of precursor solution pA and solution B.

**Table 8: The composition of the precursor solution pA and thesolution B for the preparation of the hydrogel with the content of blood 5% (v/v).**

| **Solution pA** | | **Solution B** | |
|---|---|---|---|
| HRP | 0.9 U/mL | H₂O₂ | 9.2 mmol/L |
| CaCl₂·2H₂O | 0.2 mol/L | HA-TA | 50 mg/mL |
| HA-TA | 50 mg/mL | | |
| Blood | 10 % (v/v) | | |

Hydrogel with the content 5% (v/v) of blood was prepared by mixing the solution pA and the solution B in volume ratio 1 : 1.

Thus prepared hydrogel contains enzyme horseradish peroxidase, calcium ions, covalently crosslinking hydroxyphenyl derivative of hyaluronan (crossHA-TA) and blood in following concentrations:

| | |
|---|---|
| HRP | 0.45 U/mL |
| CaCl₂·2H₂O | 0.1 mol/L |
| crossHA-TA | 50 mg/mL |
| Blood | 5 % (v/v) |

### Example 12

### Preparation of hydrogels with blood content (5% v/v)

Derivative HA-TA (Mw = 2.81 × 10⁵ g.mol⁻¹, DS 2.2 %) was used for preparation of solutions of the means for hydrogel preparation. The precursor hydrogel solution pA was prepared from solution A (1.8 mL) of the means for hydrogel preparation of composition:

| | |
|---|---|
| HRP | 0.889 U/ml |
| CaCl₂.2H₂O | 0.223 mol/L |
| HA-TA | 15.556 mg/mL, |

by addition of 0.2 ml of blood. Further, 2 ml of solution B of the means were used for hydrogel preparation. Table 9 shows composition of precursor solution pA and solution B.

**Table 9: The composition of the precursor solution pA and the solution B for the preparation of the hydrogel with the content of blood 5% (v/v).**

| **Solution pA** | | **Solution B** | |
|---|---|---|---|
| HRP | 0.8 U/mL | H₂O₂ | 4.6 mmol/L |
| CaCl₂·2H₂O | 0.2 mol/L | HA-TA | 26 mg/mL |
| HA-TA | 14 mg/mL | | |
| Blood | 10 % (v/v) | | |

Hydrogel with content 5% (v/v) of blood was prepared by mixing the solution pA and the solution B in the volume ratio 1:1.

Thus prepared hydrogel contains enzyme horseradish peroxidase, calcium ions, covalently crosslinked hydroxyphenyl derivative of hyaluronan (crossHA-TA) and blood in following concentrations:

| | |
|---|---|
| HRP | 0.4 U/mL |
| CaCl₂·2H₂O | 0.1 mol/L |
| crossHA-TA | 20 mg/mL |
| Blood | 5 % (v/v) |

### Example 13

### Preparation of hydrogels with blood content (5%)

Derivative HA-TA (Mw = 1.45 × 10⁶ g.mol⁻¹, DS 1.1 %) was used for preparation of solutions of the means for the hydrogel preparation. The precursor hydrogel solution pA was prepared from solution A (1.8 ml) of the means for hydrogel preparation of composition:

| | |
|---|---|
| HRP | 0.556 U/ml |
| CaCl₂.2H₂O | 0.223 mol/L |
| HA-TA | 11.112 mg/mL, |

by addition of 0.2 ml of blood. Further, 2 ml of the solution B of the means were used for the hydrogel preparation. Table 10 shows composition of the precursor solution pA and the solution B.

**Table 10: The composition of the precursor solution pA and the solution B for the preparation of the hydrogel with the content of blood 5% (v/v).**

| **Solution pA** | | **Solution B** | |
|---|---|---|---|
| HRP | 0.5 U/mL | H₂O₂ | 2.3 mmol/L |
| CaCl₂·2H₂O | 0.2 mol/L | HA-TA | 10 mg/mL |
| HA-TA | 10 mg/mL | | |
| Blood | 10 % (v/v) | | |

The hydrogel with the content 5% (v/v) of blood was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and blood in following concentrations:

| | |
|---|---|
| HRP | 0.25 U/mL |
| CaCl₂·2H₂O | 0.1 mol/L |
| crossHA-TA | 10 mg/mL |
| Blood | 5 % (v/v) |

### Example 14

### Preparation of the hydrogel with the blood content (15% v/v)

Derivative HA-TA (Mw = 2.81 × 10⁵ g.mol⁻¹, DS 2.2 %) was used for the preparation of solutions of the means for hydrogel preparation. The precursor hydrogel solution pA was prepared from the solution A (1.4 ml) of the means for hydrogel preparation of composition:

| | |
|---|---|
| HR | 1.143 U/ml |
| CaCl₂.2H₂O | 0.286 mol/L |
| HA-TA | 20 mg/mL, |

by addition of 0.6 ml of blood. Further, 2 ml of the solution B of the means were used for the hydrogel preparation. Table 11 shows composition of the precursor solution pA and the solution B.

**Table 11: Composition of the precursor solution pA and the solution B for the preparation of hydrogel with the content of blood 15% (v/v).**

| **Solution pA** | | **Solution B** | |
|---|---|---|---|
| HRP | 0.8 U/mL | H₂O₂ | 4.6 mmol/L |
| CaCl₂·2H₂O | 0.2 mol/L | HA-TA | 26 mg/mL |
| HA-TA | 14 mg/mL | | |
| Blood | 30 % (v/v) | | |

The hydrogel with the content 15% (v/v) of blood was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and blood in following concentrations:

| | |
|---|---|
| HRP | 0.4 U/mL |
| CaCl₂·2H₂O | 0.1 mol/L |
| crossHA-TA | 20 mg/mL |
| Blood | 15 % (v/v) |

### Example 15

### Preparation of hydrogel with blood content (30% v/v)

Derivative HA-TA (Mw = 5.4 × 10⁴ g.mol⁻¹, DS 6.2 %) was used for the preparation of solutions of the means for hydrogel preparation. The precursor hydrogel solution pA was prepared from solution A (0.8 ml) of the means for hydrogel preparation of composition:

| | |
|---|---|
| HRP | 25 U/ml |
| CaCl₂.2H2O | 0.5 mol/L |
| HA-TA | 125 mg/mL, |

by addition of 1.2 ml of blood. Further, 2 ml of solution B of the means were used for the hydrogel preparation. Table 12 shows composition of the precursor solution pA and the solution B.

**Table 12: Composition of the precursor solution pA and the solution B for the preparation of the hydrogel with the content of blood 15% (v/v).**

| **Solution pA** | | **Solution B** | |
|---|---|---|---|
| HRP | 0.9 U/mL | H₂O₂ | 9.3 mmol/L |
| CaCl₂·2H₂O | 0.2 mol/L | HA-TA | 50 mg/mL |
| HA-TA | 50 mg/mL | | |
| Blood | 60 % (v/v) | | |

Hydrogel with the content 30% (v/v) of blood was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and blood in following concentrations:

| | |
|---|---|
| HRP | 0.45 U/mL |
| CaCl₂·2H₂O | 0.1 mol/L |
| crossHA-TA | 50 mg/mL |
| Blood | 30 % (v/v) |

### Example 16

### Preparation of the hydrogel with the blood content (30 % v/v)

Derivative HA-TA (Mw = 2.81 × 10⁵ g.mol⁻¹, DS 2.2 %) was used for the preparation of solutions of the means for hydrogel preparation. The precursor hydrogel solution pA was prepared from solution A (0.8 ml) of the means for hydrogel preparation of composition:

| | |
|---|---|
| HRP | 2 U/ml |
| CaCl₂.2H₂O | 0.5 mol/L |
| HA-TA | 35 mg/mL |

by addition of 1.2 ml of blood prepared the precursor hydrogel solution pA. Further, 2 ml of the solution B of the means were used for the hydrogel preparation. Table 13 shows the composition of the precursor solution pA and the solution B.

**Table 13: Composition of the precursor solution pA and the solution B for the preparation of hydrogel with the content of blood 30% (v/v).**

| **Solution pA** | | **Solution B** | |
|---|---|---|---|
| HRP | 0.8 U/mL | H₂O₂ | 4.6 mmol/L |
| CaCl₂·2H₂O | 0.2 mol/L | HA-TA | 26 mg/mL |
| HA-TA | 14 mg/mL | | |
| Blood | 60 % (v/v) | | |

Hydrogel with the content 30% (v/v) of blood was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and blood in following concentrations:

| | |
|---|---|
| HRP | 0.4 U/mL |
| CaCl₂·2H₂O | 0.1 mol/L |
| crossHA-TA | 20 mg/mL |
| Blood | 30 % (v/v) |

### Example 17

### Preparation of the hydrogel with the blood plasma content (5 % v/v)

Derivative HA-TA (Mw = 2.81 × 10⁵ g.mol⁻¹, DS 2.2 %) was used for the preparation of solutions of the means for the hydrogel preparation. The precursor hydrogel solution pA was prepared from solution A (1.8 ml) of the means for hydrogel preparation of composition:

| | |
|---|---|
| HRP | 0.889 U/ml |
| Ca(HCO₃)₂ | 0.223 mol/L |

was by the addition of 0.2 ml of the blood. Further, 2 ml of the solution B of the means were used for the hydrogel preparation. Table 14 shows composition of the precursor solution pA and the solution B.

**Table 14: Composition of the precursor solution pA and the solution B for the preparation of hydrogel with the content of blood 5% (v/v).**

| **Solution pA** | | **Solution B** | |
|---|---|---|---|
| HRP | 0.8 U/mL | H₂O₂ | 4.6 mmol/L |
| Ca(HCO₃)₂ | 0.2 mol/L | HA-TA | 26 mg/mL |
| Blood plasma | 10 % (v/v) | | |

Hydrogel with the content 5 % (v/v) of blood plasma was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and blood plasma in following concentrations:

| | |
|---|---|
| HRP | 0.4 U/mL |
| Ca(HCO₃)₂ | 0.1 mol/L |
| crossHA-TA | 13 mg/mL |
| Blood plasma | 5 % (v/v) |

### Example 18

### Preparation of hydrogels with the blood plasma content (15% v/v)

Derivative HA-TA (Mw = 2.81 × 10⁵ g.mol⁻¹, DS 2.2 %) was used for the preparation of solutions of the means for hydrogel preparation. The precursor hydrogel solution pA was prepared from solution A (1.4 ml) of the means for hydrogel preparation of composition:

| | |
|---|---|
| HRP | 1.143 U/ml |
| CaCl₂.2H₂O | 0.286 mol/L |

by addition of 0.6 ml of blood. As the precursor solution pB were used 2 ml of the solution B of the means for hydrogel preparation. Table 15 shows composition of the precursor solution pA and solution B.

**Table 15: Composition of the precursor solution pA and the solution B for the preparation of the hydrogel with the content of blood plasma 15% (v/v).**

| **Solution pA** | | **Solution B** | |
|---|---|---|---|
| HRP | 0.8 U/mL | H₂O₂ | 4.6 mmol/L |
| CaCl₂·2H₂O | 0.2 mol/L | HA-TA | 26 mg/mL |
| Blood plasma | 30 % (v/v) | | |

Hydrogel with the content 15% (v/v) of blood plasma was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and blood plasma in following concentrations:

| | |
|---|---|
| HRP | 0.4 U/mL |
| CaCl₂·2H₂O | 0.1 mol/L |
| crossHA-TA | 13 mg/mL |
| Blood plasma | 15 % (v/v) |

### Example 19

### Preparation of the hydrogel with the blood plasma content (30% v/v)

Derivative HA-TA (Mw = 2.81 × 10⁵ g.mol⁻¹, DS 2.2 %) was used for the preparation of solutions of the means for the hydrogel preparation. The precursor hydrogel solution pA was prepared from the solution A (0.8 ml) of the means for hydrogel preparation of composition:

| | |
|---|---|
| HRP | 2 U/ml |
| CaCl₂.2H₂O | 0.5 mol/L |

by addition of 1.2 ml of blood. Further, 2 ml of the solution B of the means were used for the hydrogel preparation. Table 16 shows composition of the precursor solution pA and the solution B.

**Table 16: Composition of the precursor solution pA and the solution B for preparation of hydrogel with content of blood 30% (v/v).**

| **Solution pA** | | **Solution B** | |
|---|---|---|---|
| HRP | 0.8 U/mL | H₂O₂ | 4.6 mmol/L |
| CaCl₂·2H₂O | 0.2 mol/L | HA-TA | 26 mg/mL |
| Blood plasma | 60 % (v/v) | | |

Hydrogel with the content 30% (v/v) of blood was prepared by mixing the solution pA and the solution B in volume ratio 1 : 1.

Thus prepared hydrogel contains enzyme horseradish peroxidase, calcium ions, covalently crosslinked hydroxyphenyl derivative of hyaluronan (crossHA-TA) and blood plasma in following concentrations:

| | |
|---|---|
| HRP | 0.4 U/mL |
| CaCl₂·2H₂O | 0.1 mol/L |
| crossHA-TA | 13 mg/mL |
| Blood plasma | 30 % (v/v) |

### Example 20

### Preparation of hydrogels with the content of blood plasma fraction reaches in blood platelets (PRP, 5% v/v)

Derivative HA-TA (Mw = 2.81 × 10⁵ g.mol⁻¹, DS 2.2 %) was used for the preparation of solutions of the means for the hydrogel preparation. The precursor hydrogel solution pA was prepared from the solution A (0.8 ml) of the means for hydrogel preparation of composition:

| | |
|---|---|
| HRP | 0.889 U/ml |
| Calcium acetate | 0.223 mol/L |

by addition of 0.2 ml PRP. 2 ml of the solution B of the means were used for the hydrogel preparation. Table 17 shows composition of the precursor solution pA and the solution B.

**Table 17: Composition of the precursor solution pA and thesolution B for the preparation of hydrogel with the content of PRP 5% (v/v).**

| **Solution pA** | | **Solution B** | |
|---|---|---|---|
| HRP | 0.8 U/mL | H₂O₂. | 4.6 mmol/L |
| Calcium acetate | 0.2 mol/L | HA-TA | 26 mg/mL |
| PRP | 10 % (v/v) | | |

Hydrogel with the content 5% (v/v) of PRP was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and PRP in following concentrations:

| | |
|---|---|
| HRP | 0.4 U/mL |
| Calcium acetate | 0.1 mol/L |
| crossHA-TA | 13 mg/mL |
| PRP | 5 % (v/v) |

### Example 21

### Preparation of hydrogels with the content of blood plasma fraction reaches in blood platelets (PRP, 5% v/v)

Derivative HA-TA (Mw = 2.81 × 10⁵ g.mol⁻¹, DS 2.2 %) was used for the preparation of solutions of the means for the hydrogel preparation. The precursor hydrogel solution pA was prepared from the solution A (1.8 ml) of the means for the hydrogel preparation of composition:

| | |
|---|---|
| HRP | 0.889 U/ml |
| Calcium lactate | 0.023 mol/L |

by addition of 0.2 ml of PRP. 2 ml of the solution B of the means were used for the hydrogel preparation. Table 18 shows composition of the precursor solution pA and the solution B.

**Table 18: Composition of the precursor solution pA and the solution B for the preparation of hydrogel with the content of PRP 5% (v/v).**

| **Solution pA** | | **Solution B** | |
|---|---|---|---|
| HRP | 0.8 U/mL | H₂O₂ | 4.6 mmol/L |
| Calcium lactate | 0.02 mol/L | HA-TA | 26 mg/mL |
| PRP | 10 % (v/v) | | |

The hydrogel with the content 5% (v/v) of PRP was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and PRP in following concentrations:

| | |
|---|---|
| HRP | 0.4 U/mL |
| Calcium lactate | 0.1 mol/L |
| crossHA-TA | 13 mg/mL |
| PRP | 5 % (v/v) |

### Example 22

### Preparation of hydrogels with the content of blood plasma fraction reaches in blood platelets (PRP, 15% v/v)

Derivative HA-TA (Mw = 1.45 × 10⁶ g.mol⁻¹, DS 1.1 %) was used for the preparation of solutions of the means for the hydrogel preparation. The precursor hydrogel solution pA was prepared from solution A (1.4 ml) of the means for the hydrogel preparation of composition:

| | |
|---|---|
| HRP | 1.143 U/mL |
| CaCl₂.2H₂O | 0.286 mol/L |

by addition of 0.6 ml of PRP. 2 ml of thesolution B of the means were used for the hydrogel preparation. Table 19 shows composition of the precursor solution pA and the solution B.

**Table 19: Composition of the precursor solution pA and the solution B for the preparation of the hydrogel with the content of PRP 15% (v/v).**

| **Solution pA** | | **Solution B** | |
|---|---|---|---|
| HRP | 0.8 U/mL | H₂O₂ | 4.6 mmol/L |
| CaCl₂·2H₂O | 0,2 mol/L | HA-TA | 26 mg/mL |
| PRP | 30 % (v/v) | | |

The hydrogel with the content 15% (v/v) of PRP was prepared by mixing the solution pA and the solution B in the volume ratio 1:1.

Thus prepared hydrogel contains enzyme horseradish peroxidase, calcium ions, covalently crosslinked hydroxyphenyl derivative of hyaluronan (crossHA-TA) and PRP in following concentrations:

| | |
|---|---|
| HRP | 0.4 U/mL |
| CaCl₂·2H₂O | 0.1 mol/L |
| crossHA-TA | 13 mg/mL |
| PRP | 15 % (v/v) |

### Example 23

### Preparation of hydrogels with the content of blood plasma fraction reaches in blood platelets (PRP, 30%)

Derivative HA-TA (Mw = 2.81 × 10⁵ g.mol⁻¹, DS 2.2 %) was used for the preparation of solutions of the means for hydrogel preparation. The precursor hydrogel solution pA was prepared from the solution A (0.8 ml) of the means for the hydrogel preparation of composition:

| | |
|---|---|
| HRP | 2 U/mL |
| CaCl₂.2H₂O | 0.5 mol/L |

by addition of 1.2 ml of PRP. Further, 2 ml of the solution B of the means were used for the hydrogel preparation. Table 20 shows composition of the precursor solution pA and the solution B.

**Table 20: Composition of the precursor solution pA and the solution B for the preparation of the hydrogel with the content of PRP 30% (v/v).**

| **Solution pA** | | **Solution B** | |
|---|---|---|---|
| HRP | 0.8 U/mL | H₂O₂ | 4.6 mmol/L |
| CaCl₂·2H₂O | 0.2 mol/L | HA-TA | 26 mg/mL |
| PRP | 60 % (v/v) | | |

The hydrogel with the content 30% (v/v) of PRP was prepared by mixing the solution pA and the solution B in volume ratio 1 : 1.

Thus prepared hydrogel contains enzyme horseradish peroxidase, calcium ions, covalently crosslinked hydroxyphenyl derivative of hyaluronan (crossHA-TA) and PRP in following concentrations:

| | |
|---|---|
| HRP | 0.4 U/mL |
| CaCl₂·2H₂O | 0.1 mol/L |
| crossHA-TA | 13 mg/mL |
| PRP | 30 % (v/v) |

### Example 24

### Preparation of hydrogels with the content of blood plasma fraction reaches in blood platelets (PRP, 30)

Derivative HA-TA (Mw = 1.45 × 10⁶ g.mol⁻¹, DS 1.1 %) was used for the preparation of solutions of the means for the hydrogel preparation. The precursor hydrogel solution pA was prepared from the solution A (0.8 ml) of the means for the hydrogel preparation of composition:

| | |
|---|---|
| HRP | 2 U/mL |
| Ca(H₂PO₄)₂ | 0.05 mol/L |

by addition of 1.2 ml of PRP prepared the precursor hydrogel solution pA. Further, 2 ml of the solution B of the means were used for the hydrogel preparation. Table 21 shows composition of the precursor solution pA and the solution B.

**Table 21: Composition of the precursor solution pA and the solution B for the preparation of the hydrogel with the content of PRP 30% (v/v).**

| **Solution pA** | | **Solution B** | |
|---|---|---|---|
| HRP | 0.8 U/mL | H₂O₂ | 4.6 mmol/L |
| Ca(H₂PO₄)₂ | 0.02 mol/L | HA-TA | 26 mg/mL |
| PRP | 60 % (v/v) | | |

The hydrogel with the content 30 % (v/v) of PRP was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and PRP in following concentrations:

| | |
|---|---|
| HRP | 0.4 U/mL |
| CaCl₂·2H₂O | 0.01 mol/L |
| crossHA-TA | 13 mg/mL |
| PRP | 30 % (v/v) |

### Example 25

### Preparation of hydrogels with the content of blood plasma fraction reaches in blood platelets (PRP, 30% v/v)

Derivative HA-TA (Mw = 9.5 × 10⁴ g.mol⁻¹, DS 8.6 %) was used for the preparation of solutions of the means for the hydrogel preparation. The precursor hydrogel solution pA was prepared from the solution A (0.8 ml) of the means for the hydrogel preparation of composition:

| | |
|---|---|
| HRP | 2 U/ml |
| Ca(H₂PO₄)₂ | 0.05 mol/L |
| HA-TA | 100 mg/mL |

by addition of 1.2 ml of PRP. 2 ml of the solution B of the means were used for the hydrogel preparation. Table 22 shows composition of the precursor solution pA and the solution B.

**Table 22: Composition of the precursor solution pA and the solution B for the preparation of the hydrogel with the content of PRP 30% (v/v).**

| **Solution pA** | | **Solution B** | |
|---|---|---|---|
| HRP | 0.8 U/mL | H₂O₂ | 4.6 mmol/L |
| Ca(H₂PO₄)₂ | 0.02 mol/L | | |
| HA-TA | 40 mg/mL | | |
| Blood plasma | 60 % (v/v) | | |

The hydrogel with the content 30% (v/v) of PRP was prepared by mixing the solution pA and the solution B in the volume ratio 1 : 1.

Thus prepared hydrogel contains enzyme horseradish peroxidase, calcium ions, covalently crosslinked hydroxyphenyl derivative of hyaluronan (crossHA-TA) and PRP in following concentrations:

| | |
|---|---|
| HRP | 0.4 U/mL |
| Ca(H₂PO₄)₂ | 0.01 mol/L |
| crossHA-TA | 20 mg/mL |
| Blood plasma | 30 % (v/v) |

### Example 26

### Preparation of hydrogels with the blood content (5% v/v)

Derivative HA-TA (Mw = 2.81 × 10⁵ g.mol⁻¹, DS 2.2 %) was used for the preparation of solutions of the means for the hydrogel preparation. The precursor hydrogel solution pA was prepared from the solution A (1.8 ml) of the means for the hydrogel preparation of composition:

| | |
|---|---|
| HRP | 0.889 U/mL |
| CaCl₂.2H₂O | 0.223 mol/L |
| HA-TA | 15.556 mg/mL, |

by addition of 0.2 mL of blood. 2 mL of solution B of the means were used for the hydrogel preparation. Table 23 shows composition of the precursor solution pA and the solution B.

**Table 23: Composition of the precursor solution pA and the solution B for the preparation of the hydrogel with the content of blood 5% (v/v).**

| **Solution pA** | | **Solution B** | |
|---|---|---|---|
| HRP | 0.8 U/mL | H₂O₂ | 4.6 mmol/L |
| CaCl₂·2H₂O | 0,2 mol/L | | |
| HA-TA | 14 mg/mL | | |
| Blood | 10 % (v/v) | | |

The hydrogel with the content 5% (v/v) of PRP was prepared by mixing the solution pA and the solution B in volume ratio 1:1.

Thus prepared hydrogel contains enzyme horseradish peroxidase, calcium ions, covalently crosslinked hydroxyphenyl derivative of hyaluronan (crossHA-TA) and blood in following concentrations:

| | |
|---|---|
| HRP | 0.4 U/mL |
| CaCl₂·2H₂O | 0.1 mol/L |
| crossHA-TA | 7 mg/mL |
| blood | 5 % (v/v) |

### References

[1] Jin R. In-Situ Forming Biomimetic Hydrogels for Tissue Regeneration. In: Lin C, editor. Biomedicine: InTech; 2012.
[2] Sun S, Cao H, Su H, Tan T. Preparation and characterization of a novel injectable in situ cross-linked hydrogel. Polym Bull. 2009;62:699-711.
[3] Sklubalova Z. [In situ gelling polymers for ophthalmic drops]. Ceska Slov Farm. 2005;54:4-10.
[4] Xu X, Jha AK, Harrington DA, Farach-Carson MC, Jia X. Hyaluronic Acid-Based Hydrogels: from a Natural Polysaccharide to Complex Networks. Soft matter. 2012;8:3280-94.
[5] Stem R, Kogan G, Jedrzejas MJ, Šoltés L. The many ways to cleave hyaluronan. Biotechnology advances. 2007;25:537-57.
[6] Salwowska NM, Bebenek A, Ż 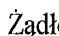 d 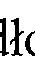 o DA, Wcis 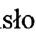 o□Dziadecka DL. Physiochemical properties and application of hyaluronic acid: a systematic review. Journal of Cosmetic Dermatology. 2016;15:520-6.
[7] Tognana E, Borrione A, De Luca C, Pavesio A. Hyalograft C: hyaluronan-based scaffolds in tissue-engineered cartilage. Cells, tissues, organs. 2007;186:97-103.
[8] Buck Ii DW, Alam M, Kim JYS. Injectable fillers for facial rejuvenation: a review. Journal of Plastic, Reconstructive & Aesthetic Surgery. 2009;62:11-8.
[9] Li P, Raitcheva D, Hawes M, Moran N, Yu X, Wang F, et al. Hylan G-F 20 maintains cartilage integrity and decreases osteophyte formation in osteoarthritis through both anabolic and anti-catabolic mechanisms. Osteoarthritis and cartilage. 2012;20:1336-46.
[10] Prestwich GD. Hyaluronic acid-based clinical biomaterials derived for cell and molecule delivery in regenerative medicine. Journal of controlled release : official journal of the Controlled Release Society. 2011;155:193-9.
[11] Burdick JA, Prestwich GD. Hyaluronic Acid Hydrogels for Biomedical Applications. Advanced Materials. 2011;23:H41-H56.
[12] Calabro A, Akst L, Alam D, Chan J, Darr AB, Fukamachi K, et al. Hydroxyphenyl cross-linked macromolecular network and applications thereof. United States: The Cleveland Clinic Foundation (Cleveland, OH, US); 2008.
[13] Kurisawa M, Lee F, Chung JE. Formation of Hydrogel in the Presence of Peroxidase and Low Concentration of Hydrogen Peroxide 2009.
[14] Lee F, Chung JE, Kurisawa M. An injectable enzymatically crosslinked hyaluronic acid-tyramine hydrogel system with independent tuning of mechanical strength and gelation rate. Soft Matter. 2008;4:880-7.
[15] Darr A, Calabro A. Synthesis and characterization of tyramine-based hyaluronan hydrogels. Journal of Materials Science: Materials in Medicine. 2009;20:33-44.
[16] Akkara JA, Senecal KJ, Kaplan DL. Synthesis and characterization of polymers produced by horseradish peroxidase in dioxane. Journal of Polymer Science Part A: Polymer Chemistry. 1991;29:1561-74.
[17] Shutava T, Zheng Z, John V, Lvov Y. Microcapsule modification with peroxidase-catalyzed phenol polymerization. Biomacromolecules. 2004;5:914-21.
[18] Ghan R, Shutava T, Patel A, John VT, Lvov Y. Enzyme-Catalyzed Polymerization of Phenols within Polyelectrolyte Microcapsules. Macromolecules. 2004;37:4519-24.
[19] Higashimura H, Kobayashi S. Oxidative Polymerization: John Wiley & Sons, Inc.; 2002.
[20] Veitch NC. Horseradish peroxidase: a modern view of a classic enzyme. Phytochemistry. 2004;65:249-59.
[21] Kurisawa M, Lee F, Wang L-S, Chung JE. Injectable enzymatically crosslinked hydrogel system with independent tuning of mechanical strength and gelation rate for drug delivery and tissue engineering. Journal of Materials Chemistry. 2010;20:5371-5.
[22] Snyder TN, Madhavan K, Intrator M, Dregalla RC, Park D. A fibrin/hyaluronic acid hydrogel for the delivery of mesenchymal stem cells and potential for articular cartilage repair. Journal of Biological Engineering. 2014;8.
[23] Li Z, Kaplan KM, Wertzel A, Peroglio M, Amit B, Alini M, et al. Biomimetic fibrin-hyaluronan hydrogels for nucleus pulposus regeneration. Regenerative medicine. 2014;9:309-26.
[24] Chance B. An intermediate compound in the catalase-hydrogen peroxide reaction. Acta chem scand. 1947;1:236-67.
[25] Odajima T, Yamazaki I. Myeloperoxidase of the leukocyte of normal blood. I. Reaction of myeloperoxidase with hydrogen peroxide. Biochimica et Biophysica Acta (BBA)-Enzymology. 1970;206:71-7.
[26] Giulivi C, Davies KJA. [30] Hydrogen peroxide-mediated ferrylhemoglobin generation in Vitro and in red blood cells. Methods in Enzymology: Academic Press; 1994. p. 490-6.
[27] Gasparro R, Qorri E, Valletta A, Masucci M, Sammartino P, Amato A, et al. Non-Transfusional Hemocomponents: From Biology to the Clinic-A Literature Review. Bioengineering. 2018;5:27.
[28] Sell SA, Ericksen JJ, Bowlin GL. The incorporation and controlled release of platelet□rich plasma□derived biomolecules from polymeric tissue engineering scaffolds. Polymer International. 2012;61:1703-9.
[29] Caroline D. Hoemann MDB, Marc D. Mckee. Composition and method for the repair and regeneration of cartilage and other tissues 2001.
[30] Ahmadi R, Bruijn JDd. Biocompatibility and gelation of chitosan-glycerol phosphate hydrogels. Journal of Biomedical Materials Research Part A. 2008;86A:824-32.
[31] Zan J, Chen H, Jiang G, Lin Y, Ding F. Preparation and properties of crosslinked chitosan thermosensitive hydrogel for injectable drug delivery systems. Journal of Applied Polymer Science. 2006;101:1892-8.
[32] Shive MS, Hoemann CD, Restrepo A, Hurtig MB, Duval N, Ranger P, et al. BST-CarGel: in situ chondroinduction for cartilage repair. Operative Techniques in Orthopaedics. 2006;16:271-8.
[33] Lee H-R, Park KM, Joung YK, Park KD, Do SH. Platelet-rich plasma loaded hydrogel scaffold enhances chondrogenic differentiation and maturation with up-regulation of CB1 and CB2. Journal of Controlled Release. 2012;159:332-7.
[34] Lee F, Kurisawa M. Formation and stability of interpenetrating polymer network hydrogels consisting of fibrin and hyaluronic acid for tissue engineering. Acta Biomaterialia. 2013;9:5143-52.

## Claims

1. Means for use in a preparation of a hydrogel, **characterized in that,** it comprises two separate solutions A and B, of which the solution A comprises enzyme horseradish peroxidase of activity 0.5 to 2.25 U/mL and the solution B comprises hydrogen peroxide at 2 to 10 mmol/L, at least one solution A or B comprising calcium ions in concentration 0.05 to 0.55 mol/L in the form of a pharmaceutically acceptable salt and further the solution A and/or B comprises hydroxyphenyl derivative of hyaluronan of a general formula I 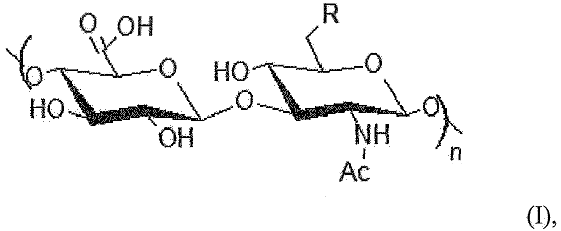 wherein n is in the range 2 to 7500 and wherein R is OH or a substituent NHR₂CONHR₁ArOH of a general formula II, 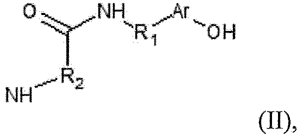 wherein Ar is phenylene and R₁ ethylene or Ar is indolylene and R₁ is ethylene or Ar is hydroxyphenylene and R₁ is carboxyethylene and R₂ is alkylene of 3 to 7 carbon atoms, in concentration 10 to 125 mg/mL.

2. Means according to claim 1, **characterized in that,** the solution A comprises enzyme horseradish peroxidase of activity preferably 1.8 to 2.2 U/mL, more preferably 1.0 to 1.3 U/mL and the solution B comprises hydrogen peroxide preferably 3 to 7 mmol/L, more preferably 4 to 5 mmol/L, at least one solution A or B comprising calcium ions in concentration preferably 0.25 to 0.55 mol/L, more preferably 0.25 to 0.45 mmol/L, most preferably 0.25 to 0.32 mmol/L and hydroxyphenyl derivative of hyaluronan of the general formula I of concentration preferably 10 to 35 mg/mL, more preferably 20 to 26 mg/mL.

3. Means according to claim 1 or claim 2, **characterized in that,** mass average molar mass of hydroxyphenyl derivative of hyaluronan of the general formula I is in the range od 4 × 10⁴ to 3 × 10⁶ g/mol, preferably 1 × 10⁵ to 1 × 10⁶ g/mol, more preferably 2 × 10⁵ to 4 × 10⁵ g/mol, the substitution degree of hydroxyphenyl derivative of hyaluronan of the general formula I is in the range 1 to 10%, preferably 2 to 5%, more preferably 2 to 4%.

4. Means according to any of claims 1 to 3, **characterized in that,** the solution A comprises:
• hydroxyphenyl derivative of hyaluronan of the general formula I of mass average molar mass in the range of 4 × 10⁴ to 3 × 10⁶ g/mol with the substitution degree 1 to 10% and the concentration 10 to 125 mg/mL,
• horseradish peroxidase of the activity 0.5 to 2.25 U/mL,
• calcium ions in the concentration 0.05 to 0.55 mol/L,
and the solution B comprises:
• hydroxyphenyl derivative of hyaluronan of the general formula I of mass average molar mass in the range of 4 × 10⁴ to 3 × 10⁶ g/mol, with the substitution degree 1 to 10% and the concentration 10 to 50 mg/mL
• hydrogen peroxide in the concentration range of 2 to 10 mmol/L.

5. Means according to any of the claims 1 to 4, **characterized in that,** the solution A comprises:
• hydroxyphenyl derivative of hyaluronan of the general formula I of mass average molar mass in the range of 1 × 10⁵ to 1 × 10⁶ g/mol, the substitution degree 2 to 5%, in the concentration 10 to 35 mg/mL.
• horseradish peroxidase of the activity 0.5 to 2.25 U/mL,
• calcium ions at the concentration 0.05 to 0.55 mol/L.
and the solution B comprises:
• hydroxyphenyl derivative of hyaluronan according to the general formula (I) of mass average molar mass in the rangeof 1 × 10⁵ to 1 × 10⁶ g/mol with the substitution degree 2 to 5 % and the concentration 26 mg/mL.
• hydrogen peroxide in the concentration range of 3 to 7 mmol/L.

6. Means according to any of claims 1 to 5, **characterized in that,** solution A comprises:
• hydroxyphenyl derivative of hyaluronan of general formula I with mass average molar mass in the range 2 × 10⁵ g/mol to 4 × 10⁵ g/mol with substitution degree 2 to 5% and concentration 20 mg/mL,
• horseradish peroxidase of activity 1.0 to 1.3 U/mL,
• calcium ions at concentration 0.25 to 0.32 mol/L
and solution B comprises:
• hydroxyphenyl derivative of hyaluronan of general formula I of mass average molar mass in the range 2 × 10⁵ g/mol to 4 × 10⁵ g/mol with substitution degree 2 to 5% and concentration 26 mg/mL,
• hydrogen peroxide in the range 4 to 5 mmol/L.

7. Means according to any of claims 1 to 3, **characterized in that,** the solution A comprises:
• horseradish peroxidase of the activity 1.8 to 2.2 U/mL,
• calcium ions at the concentration 0.45 to 0.55 mol/L
and the solution B comprises:
• hydroxyphenyl derivative of hyaluronan of the general formula I of mass average molar mass in the range of 2 × 10⁴ to 4 × 10⁵ g/mol with the substitution degree 2 to 4% and at the concentration 26 mg/mL,
• hydrogen peroxide in the concentration range of 4 to 5 mmol/L.

8. Means according to any of claims 1 to 3, **characterized in that,** the solution A comprises:
• horseradish peroxidase of the activity 0.8 to 2.2 U/mL,
• calcium ions at the concentration 0.02 to 0.5 mol/L,
• hydroxyphenyl derivative of hyaluronan of the general formula I of mass average molar mass in range of 9 × 10⁴ to 4 × 10⁵ g/mol, with the substitution degree 1 to 10% and at concentration in the range 10 to 50 mg/mL,
and solution B comprises:
• hydrogen peroxide in the concentration range of 4 to 5 mmol/L.

9. Method of a preparation of a hydrogel containing covalently crosslinked hydroxyphenyl derivative of hyaluronan, **characterized in that,** two separate solutions A and B are prepared that are defined in any of claims 1 to 8, and after that is the solution A mixed with blood or with at least one transfusion preparation with fibrinogen content giving a precursor solution A that is mixed with the solution B.

10. Method of the preparation of the hydrogel according to claim 9, **characterized in that,** 10 to 60 % (v/v) of blood or the transfusion preparation with fibrinogen content, preferably 30 to 60 % (v/v) is added into the solution A.

11. Method of the preparation of the hydrogel according to claim 9 or claim 10, **characterized in that,** the precursor solution A is mixed with the solution B in volume ratio 1:1.

12. Hydrogels prepared by method according to claims 9 to 11, **characterized in that,** they comprise enzyme horseradish peroxidase of activity 0.1 to 2.5 U/mL, preferably 0.25 to 0.45 U/mL, more preferably 0.35 to 0.45 U/mL, calcium ions at concentration 0.01 to 0.11 mol/L, preferably 0.09 to 0.11 mol/L, covalently crosslinked hydroxyphenyl derivative of hyaluronan of concentration 5 to 50 mg/mL, more preferably 13 to 20 mg/mL and blood or the transfusion preparation with fibrinogen content at the concentration 5 to 30 % (v/v), preferably 15 to 30 % (v/v).

13. Use of the hydrogel according to claim 12 for manufacturing of preparations in cosmetics, medicine, and/or regenerative medicine.

14. Use according to claim 13, where the preparation is selected from a group comprising wound dressing, preparation for soft tissue augmentation, preparations for viscosupplementation of synovial fluid, matrix for controlled drug release, fillings of tissue defects, scaffolds for tissue engineering.

15. Use according to claim 14, where the preparations comprise seeded and unseeded scaffolds for treatment of defects of joint cartilage and bone defects.

## Patentansprüche

1. Ein für die Anwendung bei der Vorbereitung von Hydrogel vorgesehenes Mittel, **dadurch gekennzeichnet, dass** es zwei getrennte Lösungen A und B umfasst, von denen die Lösung A als Enzym die Meerrettich-Peroxidase mit der im Bereich von 0,5 bis 2,25 U/ml liegenden Aktivität umfasst und die Lösung B Wasserstoffperoxid mit der im Bereich von 2 bis 10 mmol/l liegenden Konzentration umfasst, wobei wenigstens eine der Lösungen A und B ferner Kalziumionen in der Form eines pharmakologisch akzeptierbaren Salzes mit der im Bereich von 0,05 bis 0,55 mol/l liegenden Konzentration umfasst und zudem die Lösung A und/oder B ein Hydroxyphenyl-Derivat von Hyaluronan nach der allgemeinen Formel I umfasst, 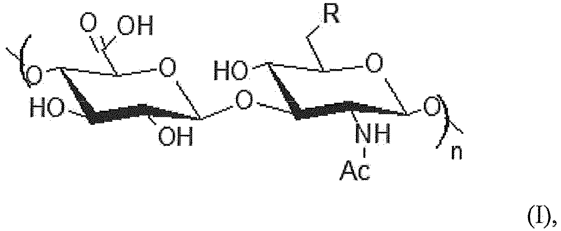 wobei n im Bereich von 2 bis 7500 liegt und wobei R eine OH-Gruppe oder ein Substituent von NHR₂CONHR₁ArOH nach der allgemeinen Formel II ist, 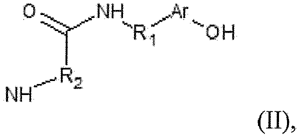 wobei Ar für Phenylen und R₁ für Ethylen steht, oder Ar für Indolylen und Ri für Ethylen steht, oder Ar für Hydroxyphenylen und Ri für Carboxyethylen steht, wobei R₂ ein Alkylen mit der im Bereich von 3 bis 7 liegenden Anzahl von Kohlenstoffatomen und mit der im Bereich von 10 bis 125 mg/ml liegenden Konzentration ist.

2. Das Mittel nach Anspruch 1, **dadurch gekennzeichnet, dass** die Lösung A als Enzym die Meerrettich-Peroxidase mit der im Bereich von 1,8 bis 2,2 U/ml, vorteilhafter im Bereich von 1,0 bis 1,3 U/ml liegenden Aktivität umfasst und die Lösung B Wasserstoffperoxid mit der im Bereich von 3 bis 7 mmol/l, vorteilhafter im Bereich von 4 bis 5 mmol/l liegenden Konzentration umfasst, wobei zumindest eine der Lösungen A oder B Kalziumionen mit der im Bereich von 0,25 bis 0,55 mol/l, vorteilhafter im Bereich von 0,25 bis 0,45 mmol/l, am vorteilhaftesten im Bereich von 0,25 bis 0,32 mmol/l liegenden Konzentration sowie ein Hydroxyphenyl-Derivat von Hyaluronan nach der allgemeinen Formel I mit der im Bereich von 10 bis 35 mg/ml, vorteilhafter im Bereich von 20 bis 26 mg/ml liegenden Konzentration umfasst.

3. Das Mittel nach Anspruch 1 oder Anspruch 2, **dadurch gekennzeichnet, dass** die gewichtsmittlere Molmasse des Hydroxyphenyl-Derivats von Hyaluronan nach der allgemeinen Formel I im Bereich von 4×10⁴ bis 3 × 10⁶ g/mol, vorteilhafter im Bereich von 1×10⁵ bis 1×10⁶ g/mol, am vorteilhaftesten im Bereich von 2×10⁵ bis 4×10⁵ g/mol liegt und der Substitutionsgrad des Hydroxyphenyl-Derivats von Hyaluronan nach der allgemeinen Formel I im Bereich von 1 bis 10 %, vorteilhafter im Bereich von 2 bis 5 %, vorteilhafter im Bereich von 2 bis 4 % liegt.

4. Das Mittel nach einem der Ansprüche 1 bis 3, **dadurch gekennzeichnet, dass** die Lösung A umfasst:
• ein Hydroxyphenyl-Derivat von Hyaluronan nach der allgemeinen Formel I mit einer im Bereich von 4 ×10⁴ bis 3 ×10⁶ g/mol liegenden gewichtsmittleren Molmasse, mit einem im Bereich von 1 bis 10 % liegenden Substitutionsgrad, sowie mit der im Bereich von 10 bis 125 mg/ml liegenden Konzentration,
• Meerrettich-Peroxidase mit der im Bereich von 0,5 bis 2,25 U/ml liegenden Aktivität,
• Kalziumionen mit der im Bereich von 0,05 bis 0,55 mol/l liegenden Konzentration,
und die Lösung B umfasst:
• ein Hydroxyphenyl-Derivat von Hyaluronan nach der allgemeinen Formel I mit der im Bereich von 4 ×10⁴ bis 3 ×10 ⁶ g/mol liegenden gewichtsmittleren Molmasse, mit dem im Bereich von 1 bis 10 % liegenden Substitutionsgrad, sowie mit der im Bereich von 10 bis 50 mg/ml liegenden Konzentration,
• Wasserstoffperoxid mit der im Bereich von 2 bis 10 mmol/l liegenden Konzentration.

5. Das Mittel nach einem der Ansprüche 1 bis 4, **dadurch gekennzeichnet, dass** die Lösung A umfasst:
• ein Hydroxyphenyl-Derivat von Hyaluronan nach der allgemeinen Formel I mit der im Bereich von 1 ×10 ⁵ bis 1 ×10 ⁶ g/mol liegenden gewichtsmittleren Molmasse, mit dem im Bereich von 2 bis 5 % liegenden Substitutionsgrad, sowie mit der im Bereich von 10 bis 35 mg/ml liegenden Konzentration,
• Meerrettich-Peroxidase mit der im Bereich von 0,5 bis 2,25 U/ml liegenden Aktivität,
• Kalziumionen mit der im Bereich von 0,05 bis 0,55 mol/l liegenden Konzentration,
und die Lösung B umfasst:
• ein Hydroxyphenyl-Derivat von Hyaluronan nach der allgemeinen Formel (I) mit einer im Bereich von 1 ×10⁵ bis 1 ×10⁶ g/mol liegenden gewichtsmittleren Molmasse, mit einem im Bereich von 2 bis 5 % liegenden Substitutionsgrad, sowie mit der Konzentration von 26 mg/ml,
• Wasserstoffperoxid mit der im Bereich von 3 bis 7 mmol/l liegenden Konzentration.

6. Das Mittel nach einem der Ansprüche 1 bis 5, **dadurch gekennzeichnet, dass** die Lösung A umfasst:
• ein Hydroxyphenyl-Derivat von Hyaluronan nach der allgemeinen Formel I mit der im Bereich von 2 ×10⁵ bis 4 ×10⁵ g/mol liegenden gewichtsmittleren Molmasse, mit dem im Bereich von 2 bis 5 % liegenden Substitutionsgrad, sowie mit der Konzentration von 20 mg/ml,
• Meerrettich-Peroxidase mit der im Bereich von 1,0 bis 1,3 U/ml liegenden Aktivität,
• Kalziumionen mit der im Bereich von 0,25 bis 0,32 mol/l liegenden Konzentration,
und die Lösung B umfasst:
• ein Hydroxyphenyl-Derivat von Hyaluronan nach der allgemeinen Formel I mit der im Bereich von 2 ×10⁵ bis 4 ×10⁵ g/mol liegenden gewichtsmittleren Molmasse, mit dem im Bereich von 2 bis 5 % liegenden Substitutionsgrad, sowie mit der Konzentration von 26 mg/ml,
• Wasserstoffperoxid mit der im Bereich von 4 bis 5 mmol/l liegenden Konzentration.

7. Das Mittel nach einem der Ansprüche 1 bis 3, **dadurch gekennzeichnet, dass** die Lösung A umfasst:
• Meerrettich-Peroxidase mit der im Bereich von 1,8 bis 2,2 U/ml liegenden Aktivität,
• Kalziumionen mit der im Bereich von 0,45 bis 0,55 mol/l liegenden Konzentration,
und die Lösung B umfasst:
• ein Hydroxyphenyl-Derivat von Hyaluronan nach der allgemeinen Formel I mit der im Bereich von 2×10⁴ bis 4 ×10⁵ g/mol liegenden gewichtsmittleren Molmasse, mit dem im Bereich von 2 bis 4 % liegenden Substitutionsgrad, sowie mit der Konzentration von 26 mg/ml,
• Wasserstoffperoxid mit der im Bereich von 4 bis 5 mmol/l liegenden Konzentration.

8. Das Mittel nach einem der Ansprüche 1 bis 3, **dadurch gekennzeichnet, dass** die Lösung A umfasst:
• Meerrettich-Peroxidase mit der im Bereich von 0,8 bis 2,2 U/ml liegenden Aktivität,
• Kalziumionen mit der im Bereich von 0,02 bis 0,5 mol/l liegenden Konzentration,
• ein Hydroxyphenyl-Derivat von Hyaluronan nach der allgemeinen Formel I mit einer im Bereich von 9 ×10⁴ bis 4 ×10⁵ g/mol liegenden gewichtsmittleren Molmasse, mit einem im Bereich von 1 bis 10 % liegenden Substitutionsgrad, sowie mit der im Bereich von 10 bis 50 mg/ml liegenden Konzentration,
und die Lösung B umfasst:
• Wasserstoffperoxid mit der im Bereich von 4 bis 5 mmol/l liegenden Konzentration.

9. Ein Verfahren zur Vorbereitung eines Hydrogels, das ein kovalent vernetztes Hydroxyphenyl-Derivat von Hyaluronan umfasst, **dadurch gekennzeichnet, dass** zwei getrennte, in einem der Ansprüche 1 bis 8 definierte Lösungen A und B vorbereitet werden, worauf die Lösung A mit Blut oder mit wenigstens einem für Transfusion vorgesehenen, Fibrinogen enthaltenden Präparat unter Bildung der als Präkursor vorhandenen Lösung A vermischt wird und die letztere anschliessend mit der Lösung B vermischt wird.

10. Das Verfahren zur Vorbereitung des Hydrogels nach Anspruch 9, **dadurch gekennzeichnet, dass** der Lösung A Blut oder ein für Transfusion vorgesehenes, Fibrinogen enthaltendes Präparat in einem Mengenanteil von 10 bis 60 % (Vol./Vol.), vorteilhaft von 30 bis 60 % (Vol./Vol.), zugegeben wird.

11. Das Verfahren zur Vorbereitung des Hydrogels nach Anspruch 9 oder Anspruch 10, **dadurch gekennzeichnet, dass** die Vermischung der als Präkursor vorhandenen Lösung A mit der Lösung B in dem Volumenverhältnis von 1:1 erfolgt.

12. Hydrogele, die in dem Verfahren nach Ansprüchen 9 bis 11 vorbereitet sind, **dadurch gekennzeichnet, dass** sie als Enzym die Meerrettich-Peroxidase mit der im Bereich von 0,1 bis 2,5 U/ml, vorteilhaft im Bereich von 0,25 bis 0,45 U/ml, vorteilhafter im Bereich von 0,35 bis 0,45 U/ml liegenden Aktivität, Kalziumionen mit der im Bereich von 0,01 bis 0,11 mol/l, vorteilhaft im Bereich von 0,09 bis 0,11 mol/l liegenden Konzentration, ein kovalent vernetztes Hydroxyphenyl-Derivat von Hyaluronan mit der im Bereich von 5 bis 50 mg/ml, vorteilhafter im Bereich von 13 bis 20 mg/ml liegenden Konzentration, sowie Blut oder ein für Transfusion vorgesehenes, Fibrinogen enthaltendes Präparat mit der im Bereich von 5 bis 30 % (Vol./Vol.), vorteilhaft im Bereich von 15 bis 30 % (Vol./Vol.) liegenden Konzentration umfasst.

13. Anwendung des Hydrogels nach Anspruch 12 zur Herstellung von Präparaten auf den Gebieten Kosmetik und Medizin, einschliesslich regenerativer Medizin.

14. Anwendung nach Anspruch 13, wobei das Präparat aus der Präparatengruppe ausgewählt ist, die Wundenabdeckungen, Präparate zur Augmentation von Weichgeweben, Präparate zur Viskosupplementierung von Synovialflüssigkeiten, Matrizen zur kontrollierten Freisetzung von Arzneien, Präparate zur Füllung von Gewebedefekten und Gerüste für das Gebiet der Gewebezüchtung umfasst.

15. Anwendung nach Anspruch 14, wobei die Präparate angesäte sowie nicht angesäte Gerüste umfassen, die zur Heilung von Gelenkknorpel- und Knochendefekten vorgesehen sind.

## Revendications

1. Moyens à utiliser dans une préparation d'un hydrogel, **caractérisés en ce qu'**ils comprennent deux solutions distinctes A et B, où la solution A comprend un enzyme de peroxydase de raifort d'une activité de 0.5 à 2.25 U/mL et la solution B comprend du peroxyde d'hydrogène de 2 à 10 mmol/L, au moins une solution A ou B comprenant des ions calcium dans une concentration de 0.05 à 0.55 mol/L dans la forme d'un sel pharmaceutiquement acceptable et en plus la solution A et/ou B comprend du dérivé d'hydroxyphényle d'hyaluronane selon une formule générale I 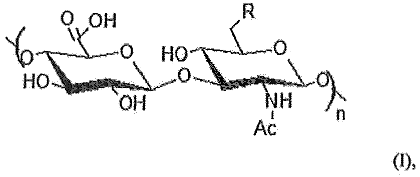 où n est dans la plage de 2 à 7500 et où R est OH ou un substituant NHR₂CONHR₁ArOH selon une formule générale II 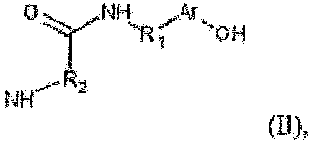 où Ar est phénylène et R₁ est éthylène ou Ar est indolylène et Ri est éthylène ou Ar est hydroxyphénylène et Ri est carboxyéthylène et R₂ est alkylène de 3 à 7 atomes de carbone, dans une concentration de 10 à 125 mg/mL.

2. Moyens selon la revendication 1, **caractérisés en ce que** la solution A comprend un enzyme de peroxydase de raifort d'une activité préférablement de 1.8 à 2.2 U/mL, plus préférablement de 1.0 à 1.3 U/mL et la solution B comprend du peroxyde d'hydrogène préférablement de 3 à 7 mmol/L, plus préférablement de 4 à 5 mmol/L, au moins une solution A ou B comprenant des ions calcium dans une concentration préférablement de 0.25 a 0.55 mol/L, plus préférablement de 0.25 à 0.45 mmol/L, le plus préférablement de 0.25 à 0.32 mmol/L, et du dérivé d'hydroxyphényle d'hyaluronane selon la formule générale I d'une concentration préférablement de 10 à 35 mg/mL, plus préférablement de 20 à 26 mg/mL.

3. Moyens selon la revendication 1 ou 2, **caractérisés en ce que** la masse molaire moyenne en poids du dérivé d'hydroxyphényle d'hyaluronane selon la formule générale I est dans la plage de 4 × 10⁴ à 3 × 10⁶ g/mol, préférablement de 1× 10⁵ à 1 × 10⁶ g/mol, plus préférablement 2 × 10⁵ à 4 × 10⁵ g/mol, le degré de substitution du dérivé d'hydroxyphényle d'hyaluronane selon la formule générale I est dans la plage de 1 à 10%, préférablement de 2 à 5%, plus préférablement de 2 à 4%.

4. Moyens selon l'une quelconque des revendications 1 à 3, **caractérisés en ce que** la solution A comprend :
• du dérivé d'hydroxyphényle d'hyaluronane selon la formule générale I de la masse molaire moyenne en poids dans la plage de 4 × 10⁴ à 3 × 10⁶ g/mol avec le degré de substitution de 1 à 10 % et la concentration de 10 à 125 mg/mL,
• de la peroxydase de raifort de l'activité de 0.5 à 2.25 U/mL,
• des ions de calcium dans la concentration de 0.05 à 0.55 mol/L,
et la solution B comprend :
• du dérivé d'hydroxyphényle d'hyaluronane selon la formule générale I de la masse molaire moyenne en poids dans la plage de 4 × 10⁴ à 3 × 10⁶ g/mol, avec le degré de substitution de 1 à 10% et la concentration de 10 à 50 mg/mL
• du peroxyde d'hydrogène dans la plage de concentration de 2 à 10 mmol/L.

5. Moyens selon l'une quelconque des revendications 1 à 4, **caractérisés en ce que** la solution A comprend :
• du dérivé d'hydroxyphényle d'hyaluronane selon la formule générale I de la masse molaire moyenne en poids dans la plage de 1 × 10⁵ à 1 × 10⁶ g/mol, le degré de substitution de 2 à 5%, dans la concentration de 10 à 35 mg/mL,
• de la peroxydase de raifort de l'activité de 0.5 à 2.25 U/mL,
• des ions de calcium dans la concentration de 0.05 à 0.55 mol/L,
et la solution B comprend :
• du dérivé d'hydroxyphényle d'hyaluronane selon la formule générale (I) de la masse molaire moyenne en poids dans la plage de 1 × 10⁵ à 1 × 10⁶ g/mol avec le degré de substitution de 2 à 5 % et la concentration de 26 mg/mL.
• du peroxyde d'hydrogène dans la plage de concentration de 3 à 7 mmol/L.

6. Moyens selon l'une quelconque des revendications 1 à 5, **caractérisés en ce que** la solution A comprend :
• du dérivé d'hydroxyphényle d'hyaluronane selon la formule générale I avec la masse molaire moyenne en poids dans la plage de 2 × 10⁵ g/mol à 4 × 10⁵ g/mol avec le degré de substitution de 2 à 5% et la concentration de 20 mg/mL,
• de la peroxydase de raifort de l'activité de 1.0 à 1.3 U/mL,
• des ions de calcium dans la concentration de 0.25 à 0.32 mol/L
et la solution B comprend :
• du dérivé d'hydroxyphényle d'hyaluronane selon la formule générale 1 de la masse molaire moyenne en poids dans la plage de 2 × 10⁵ à 4 × 10⁵ g/mol avec le degré de substitution de 2 à 5% et la concentration de 26 mg/mL,
• du peroxyde d'hydrogène dans la plage de 4 à 5 mmol/L.

7. Moyens selon l'une quelconque des revendications 1 à 3, **caractérisés en ce que** la solution A comprend :
• de la peroxydase de raifort de l'activité de 1.8 à 2.2 U/mL,
• des ions calcium dans la concentration de 0.45 à 0.55 mol/L,
et la solution B comprend :
• du dérivé d'hydroxyphényle d'hyaluronane selon la formule générale I de la masse molaire moyenne en poids dans la plage de 2 × 10⁴ à 4 × 10⁵ g/mol avec le degré de substitution de 2 à 4% et la concentration de 26 mg/mL,
• du peroxyde d'hydrogène dans la plage de concentration de 4 à 5 mmol/L.

8. Moyens selon l'une quelconque des revendications 1 à 3, **caractérisés en ce que** la solution A comprend :
• de la peroxydase de raifort de l'activité de 0.8 à 2.2 U/mL,
• des ions calcium dans la concentration de 0.02 à 0.5 mol/L,
• du dérivé d'hydroxyphényle d'hyaluronane de la formule générale I de la masse molaire moyenne en poids dans la plage de 9 × 10⁴ à 4 × 10⁵ g/mol, avec le degré de substitution de 1 à 10% et la concentration dans la plage de 10 à 50 mg/mL,
et la solution B comprend :
• du peroxyde d'hydrogène dans la plage de concentration de 4 à 5 mmol/L.

9. Procédé de préparation d'un hydrogel comprenant du dérivé d'hydroxyphényle d'hyaluronane réticulé de manière covalente, **caractérisé en ce que** deux solutions distinctes A et B sont préparées, définies selon l'une quelconque des revendications 1 à 8, et ensuite la solution A est mélangée avec du sang ou avec au moins une préparation de transfusion contenant du fibrinogène, donnant une solution A de précurseur qui est mélangée avec la solution B.

10. Procédé de préparation de l'hydrogel selon la revendication 9, **caractérisé en ce que** 10 à 60 % (v/v) de sang ou de préparation de transfusion contenant du fibrinogène, préférablement de 30 à 60 % (v/v) est ajouté à la solution A.

11. Procédé de préparation de l'hydrogel selon la revendication 9 ou 10, **caractérisé en ce que** la solution A de précurseur est mélangée avec la solution B dans un rapport volumique 1:1.

12. Hydrogels préparés au moyen du procédé selon l'une quelconque des revendications 9 à 11, **caractérisés en ce qu'**ils comprennent un enzyme de peroxydase de raifort de l'activité de 0.1 à 2.5 U/mL, préférablement de 0.25 à 0.45 U/mL, plus préférablement de 0.35 à 0.45 U/mL, des ions calcium dans la concentration de 0.01 à 0.11 mol/L, préférablement de 0.09 à 0.11 mol/L, du dérivé d'hydroxyphényle d'hyaluronane réticulé de manière covalente dans la concentration de 5 à 50 mg/mL, plus préférablement de 13 à 20 mg/mL et du sang ou de la préparation de transfusion contenant du fibrinogène dans la concentration de 5 à 30 % (v/v), préférablement de 15 à 30 % (v/v).

13. Utilisation de l'hydrogel selon la revendication 12 pour fabriquer des préparations en cosmétique, médecine, et/ou médecine régénérative.

14. Utilisation selon la revendication 13, où la préparation est sélectionnée dans un groupe comprenant des pansements, des préparations pour l'augmentation des tissus mous, des préparations pour la viscosupplémentation du liquide synovial, une matrice pour la libération contrôlée de médicaments, des remplissages de défauts tissulaires, des échafaudages pour l'ingénierie tissulaire.

15. Utilisation selon la revendication 14, où les préparations comprennent des échafaudages ensemencés ou non-ensemencés pour le traitement de défauts du cartilage articulaire et de défauts osseux.
